# EUROPEAN PATENT APPLICATION

(11) **EP 4 248 987 A1**
(43) Date of publication of application: **27.09.2023**
(21) Application number: 22163290.4
(22) Date of filing: 21.03.2022
(51) Int. Cl.: A61K 38/16, C07K 14/21

(54) **CHIMERIC BACTERIOCINS AND METHOD FOR THE CONTROL OF PSEUDOMONAS**

(71) Applicant: Nomad Bioscience GmbH, 80333 München (DE); UAB Nomads, 01112 Vilnius (LT)
(72) Inventor: PASKEVICIUS, Sarunas, 01112 Vilnius (LT); MISIUNAS, Audrius, 01112 Vilnius (LT); RAZANSKIENE, Ausra, 01112 Vilnius (LT)
(74) Representative: Blodig, Wolfgang

(57) **Abstract**

The invention provides an antibacterial protein (bacteriocin) for the control of *Pseudomonas,* preferably of *Pseudomonas aeruginosa,* and to a nucleic acid molecule encoding the bacteriocin. The invention also relates to a composition, notably a pharmaceutical composition, comprising the bacteriocin or a combination of the bacteriocins. The invention further relates to the bacteriocin or composition for use in therapy.

## Description

### FIELD OF THE INVENTION

The invention relates to an antibacterial protein (bacteriocin) for the control of *Pseudomonas,* preferably of *Pseudomonas aeruginosa,* and to a nucleic acid molecule encoding the bacteriocin. The invention also relates to a composition, notably a pharmaceutical composition, comprising the bacteriocin or a combination of the bacteriocins. The invention further relates to the bacteriocin or composition for use in therapy, preferably for the treatment of lung infection or keratitis. The invention further relates to a method of treating or preventing bacterial infection in a mammal, preferably in a human.

### BACKGROUND OF THE INVENTION

*Pseudomonas aeruginosa (P. aeruginosa)* are opportunistic Gram negative pathogenic bacteria, causing both acute and chronic infections. They harbor in their genome a large arsenal of virulence factors and antibiotic resistance determinants, conferring remarkable metabolic flexibility and the ability to adapt to multiple conditions, including the host immune response¹. Furthermore, rapid development of resistance to previously effective antimicrobials, such as fluoroquinolones, aminoglycosides, and polymyxins, has been observed². This is of particular concern for conditions due to the biofilm colonization of lungs in cystic fibrosis patients, urinary tract infections, and ear and eye infections. The involvement of *P*. *aeruginosa* in a wide range of biofilm-related infections often leads to treatment failures³. Thus, new antimicrobial substances are urgently needed.

The possibility to use bacteriocins as new generation antimicrobials has been suggested previously⁴. *P. aeruginosa* produce colicin-type bacteriocins, pyocins, belonging to different classes: deoxyribonucleases, ribonucleases, pore-forming proteins, peptidoglycan synthesis-blocking proteins, lectin-like proteins, and bacteriophage tail-like protein complexes^{5,6}. Several studies have described the use of natural pyocins for successful treatment of *P*. *aeruginosa* infections in various animal models⁷⁻¹².

The main hurdles in the clinical development of bacteriocin antimicrobials are the proteinaceous nature of bacteriocins and their limited activity spectrum. Being proteins, colicin type bacteriocins are expected to present the same challenges observed in the clinical development of bacteriophage lysins when used intravenously: short half-life, immunogenicity and weakened activity in serum^{13,14}. These problems might be less relevant in the development of topical, oral or inhaled antimicrobials.

Previously, we successfully expressed in a plant transient expression system six different pyocins¹¹. The only so far known *P. aeruginosa* pore-forming pyocin, S5, was found to be active against 40% of tested clinical isolates. S5 demonstrated superior activity compared to all other pyocins in reducing bacterial numbers in liquid culture and biofilm assays and also was most efficacious in protecting *G. mellonella* larvae from death due to *P. aeruginosa* infection¹¹.

Self-inhibition of S5-producing strains was prevented by co-expression of an immunity gene, located downstream of the bacteriocin gene. Lethality due to pore formation by pyocin S5 was transiently impeded along the secretory route via a membrane-integrated immunity protein consisting of three transmembrane helices (TMHs)¹⁵.

A general problem with bacteriocins in their limited breadth of activity or, in other words, quite high specificity against target strains or species of *Pseudomonas,* or against strains of *P. aeruginosa,* which is problematic if the pathogenic agent that causes an infection is not known or has not been identified, yet.

Therefore, it is an object of the present invention to provide bacteriocins active against *Pseudomonas* for the control of *Pseudomonas.* Further, it is an object to provide bacteriocins having a broad activity range against multiple strains of *Pseudomonas.* It is a further object to provide a remedy against infection by *Pseudomonas,* notably by *P*. *aeruginosa.*

### SUMMARY OF THE INVENTION

These objects are accomplished by:
1) A (chimeric) antibacterial protein comprising a polypeptide comprising a first polypeptide segment and, preferably contiguous thereto, a second polypeptide segment, said first polypeptide segment being selected from the following group (a-i) to (d-i), said second polypeptide segment being selected from the following group (a-i)' to (d-iii)':
   group (a-i) to (d-i):
      (a-i) the segment of the amino acid sequence of SEQ ID NO: 1 (*translocation* + *receptor binding domain of S5*), or
      (b-i) a segment having at least 80%, more preferably at least 85%, even more preferably at least 90%, and even more preferably at least 95% sequence identity to the amino acid sequence of SEQ ID NO: 1, or
      (c-i) a segment having at least 85%, preferably at least 90%, more preferably at least 95%, and most preferably at least 98 % sequence similarity to the amino acid sequence of SEQ ID NO: 1, or
      (d-i) a segment having from 1 to 60, preferably from 1 to 45, more preferably from 1 to 30, and most preferably from 1 to 15 amino acid residue substitutions, additions, insertions and/or deletions compared to the amino acid sequence of SEQ ID NO: 1;
   group (a-i)' to (d-iii)':
      (a-i)' the segment of the amino acid sequence of SEQ ID NO: 3 *(cytotoxicity domain of PmnH*), or
      (a-ii)' the segment of the amino acid sequence of SEQ ID NO: 7 *(cytotoxicity domain of Pflu095)*, or
      (a-iii)' the segment of the amino acid sequence of SEQ ID NO: 11 (*cytotoxicity domain of Pflu373*); or
      (b-i)' a segment having at least 80%, more preferably at least 85%, even more preferably at least 90%, and most preferably at least 95% sequence identity to the amino acid sequence of SEQ ID NO: 3, or
      (b-ii)' a segment having at least 80%, preferably at least 85%, more preferably at least 90%, and most preferably at least 95% sequence identity to the amino acid sequence of SEQ ID NO:7, or
      (b-iii)' a segment having at least 80%, preferably at least 85%, more preferably at least 90%, and most preferably at least 95% sequence identity to the amino acid sequence of SEQ ID NO:11; or
      (c-i)' a segment having at least 85%, preferably at least 90%, and more preferably at least 95%, and most preferably at most 98% sequence similarity to the amino acid sequence of SEQ ID NO: 3, or
      (c-ii)' a segment having at least 85%, preferably at least 90%, and more preferably at least 95%, and most preferably at most 98% sequence similarity to the amino acid sequence of SEQ ID NO: SEQ ID NO: 7, or
      (c-iii)' a segment having at least 85%, preferably at least 90%, and more preferably at least 95%, and most preferably at most 98% sequence similarity to the amino acid sequence of SEQ ID NO: SEQ ID NO: 11; or
      (d-i)' a segment having from 1 to 38, preferably from 1 to 28, more preferably from 1 to 19, and most preferably from 1 to 10 amino acid residue substitutions, additions, insertions and/or deletions compared to the amino acid sequence of SEQ ID NO: 3, or
      (d-ii)' a a segment having from 1 to 38, preferably from 1 to 28, more preferably from 1 to 19, and most preferably from 1 to 10 amino acid residue substitutions, additions, insertions and/or deletions compared to the amino acid sequence of SEQ ID NO: 7, or,
      (d-iii)' a segment having from 1 to 38, preferably from 1 to 28, more preferably from 1 to 19, and most preferably from 1 to 10 amino acid residue substitutions, additions, insertions and/or deletions compared to the amino acid sequence of SEQ ID NO: 11.
2) A (chimeric) antibacterial protein comprising a polypeptide comprising or consisting of:
   (A-i) the amino acid sequence of SEQ ID NO: 5 (*sequence of entire protein S5-PmnH*)*,* or
   (A-ii) the amino acid sequence of SEQ ID NO: 9 *(sequence of entire protein S5-Pflu095*)*,* or
   (A-iii) the amino acid sequence of SEQ ID NO: 13 *(sequence of entire protein S5-Pflu373*); or
   (B-i) an amino acid sequence having at least 80%, more preferably at least 85%, even more preferably at least 90%, and even more preferably at least 95% sequence identity to the amino acid sequence of SEQ ID NO: 5, or
   (B-ii) an amino acid sequence having at least 80%, more preferably at least 85%, even more preferably at least 90%, and even more preferably at least 95% sequence identity to the amino acid sequence of SEQ ID NO: 9, or
   (B-iii) an amino acid sequence having at least 80%, more preferably at least 85%, even more preferably at least 90%, and even more preferably at least 95% sequence identity to the amino acid sequence of SEQ ID NO: 13; or
   (C-i) an amino acid sequence having at least 85%, preferably at least 90%, and more preferably at least 95%, and most preferably at least 98% sequence similarity to the amino acid sequence of SEQ ID NO: 5, or
   (C-ii) an amino acid sequence having at least 85%, preferably at least 90%, and more preferably at least 95%, and most preferably at least 98% sequence similarity to the amino acid sequence of SEQ ID NO: 9, or
   (C-iii) an amino acid sequence having at least 85%, preferably at least 90%, and more preferably at least 95%, and most preferably at least 98% sequence similarity to the amino acid sequence of SEQ ID NO: 13; or
   (D-i) an amino acid sequence having from 1 to 100, preferably from 1 to 75, more preferably from 1 to 50, even more preferably from 1 to 25, and most preferably from 1 to 12 amino acid residue substitutions, additions, insertions and/or deletions to the amino acid sequence of SEQ ID NO: 5, or
   (D-ii) an amino acid sequence having from 1 to 100, preferably from 1 to 75, more preferably from 1 to 50, even more preferably from 1 to 25, and most preferably from 1 to 12 amino acid residue substitutions, additions, insertions and/or deletions to the amino acid sequence of SEQ ID NO: 9; or
   (D-iii) an amino acid sequence having from 1 to 100, preferably from 1 to 75, more preferably from 1 to 50, even more preferably from 1 to 25, and most preferably from 1 to 12 amino acid residue substitutions, additions, insertions and/or deletions to the amino acid sequence of SEQ ID NO: 13.
3) The antibacterial protein according to item 1, wherein said first segment is N-terminal to said second segment in the amino acid sequence of said antibiotic protein.
4) A composition comprising a protein according to any one of items 1 to 3.
5) The composition according to item 4, further comprising a second bacteriocin according to any one of items 1 to 3.
6) A pharmaceutical composition comprising an antibacterial protein as defined in any one of items 1 to 3 or comprising a composition according to any one of items 4 and 5, and one or more pharmaceutically acceptable carrier(s) or excipient(s).
7) The pharmaceutical composition according to item 6, which is a sterile aqueous solution comprising said antibacterial protein or is a solid formulation, preferably said solid formulation is a powder suitable for reconstitution in an aqueous liquid medium or is a solid formulation suitable for administration as an aerosol.
8) The antibacterial protein according to any one of items 1 to 3, or a composition according to any one of items 4 to 7 for use in therapy.
9) The antibacterial protein according to any one of items 1 to 3, or a composition according to any one of items 4 to 7, for use in the treatment or prevention of a bacterial infection, preferably of a bacterial infection by *Pseudomonas,* more preferably by *P. aeruginosa.*
10) The antibacterial protein according to any one of items 1 to 3, or a composition according to any one of items 4 to 7, for use in therapy, preferably for the treatment of lung infection or keratitis.
11) The antibacterial protein according to any one of items 1 to 3, or a composition according to any one of items 4 to 7, for use in the treatment of lung infection by administration into the lungs of a mammal as a solid or liquid aerosol; or for the treatment of keratitis by administration into an affected eye of a mammal in the form of an aqueous solution.
12) A method of treating or preventing bacterial infection in a mammal, comprising administering to a mammal in need thereof an antibacterial protein as defined in any one of items 1 to 3, or a composition according to any one of items 4 to 6, or a pharmaceutical composition according to item 7.
13) The method according to item 12, wherein bacterial lung infection is treated by administration into the lungs of a mammal as a solid or liquid aerosol; or wherein bacterial keratitis is treated by administration into an affected eye of a mammal in the form of an aqueous solution.
14) A nucleic acid molecule comprising a nucleic acid sequence encoding a protein according to any one of items 1 to 3.
15) The nucleic acid molecule according to item 14, said nucleic acid sequence comprising a polynucleotide according to any one of SEQ ID NOs: 6, 10, or 14.
16) Bacterial or eukaryotic cell comprising a nucleic acid molecule according to item 14 or 15.

The inventors have considered ways of broadening the spectrum of activity of anti-*Pseudomonas* bacteriocins, such as pyocin S5. The inventors have surprisingly found that the spectrum of activity of pyocin S5 can be broadened by modifying its killing domain to avoid its recognition by the immunity protein of the target pathogen. Other *Pseudomonas* species (in particular *P. fluorescens*) harbour in their genomes a variety of genes coding for pore forming bacteriocin-like proteins^{6,16}. Through the mining of GenBank database, the inventors retrieved several sequences of putative pore-forming bacteriocins from different *Pseudomonas* species and selected six colicin E1-like and colicin A-like proteins. The inventors then used the cytotoxic pore forming domains of these bacteriocins to construct chimeric proteins fusing them to the receptor-binding and translocation domains of pyocin S5. The inventors tested the activity of the resulting chimeric bacteriocins *in vitro* and, surprisingly, could identify bacteriocins with high cytotoxic activity and broadened specificity against *Pseudomonas* strains. The inventors found that the identified chimeric bacteriocins are active in different animal disease models, *P. aeruginosa* keratitis and lung colonization murine models, using topical and inhaled administration of the chimeric bacteriocins of the invention.

### BRIEF DESCRIPTION OF THE FIGURES

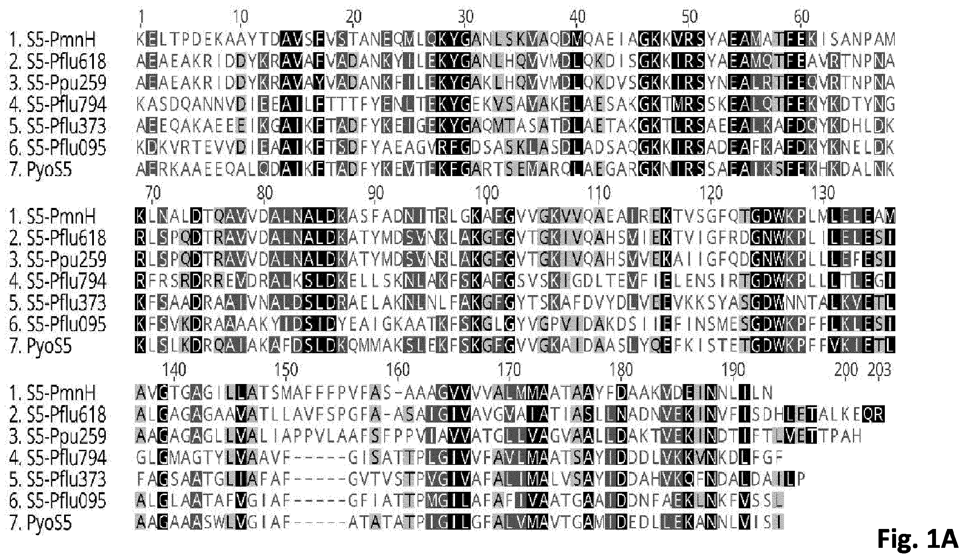

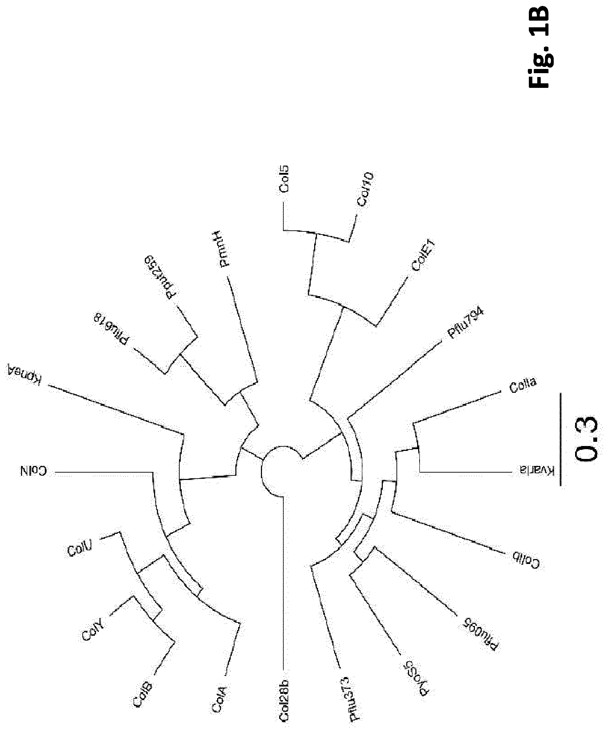

**Fig. 1** Sequence analysis of pore forming domains (pfam01024) of putative *Pseudomonas* bacteriocins. **A.** ClustalW amino acid sequence alignment of *Pseudomonas* bacteriocins with pyocin S5. The sequences shown, from top to bottom, are those of **SEQ ID NOs: 3, 19, 23, 15, 11, 7,** and **39,** respectively. **B.** Neighbor-Joining tree alignment of known *E. coli, Klebsiella* bacteriocins, pyocin S5, PmnH and putative *P. fluorescens and P. putida* bacteriocins. Col28B (CAA44310.1), ColE1 (AAA87379.1), Col10 (CAA57998.1), Col5 (CAA61102.1), Collb (AAA23188.1), Colla (WP_001283344.1), CoIN (P08083.1), ColA (P04480.1), ColU (CAA72509.1), ColB (P05819.3), ColY (AAF82683.1), KpneA (SAV78255.1), Kvarla (KDL88409), Pyocin S5 (WP_003115311), PmnH (EIK72868.1), Pflu618 (WP_034155618), Ppu259 (WP_098964259), Pflu794 (WP_081041794), Pflu373 (WP_014717373), Pflu095 (WP_016979095).

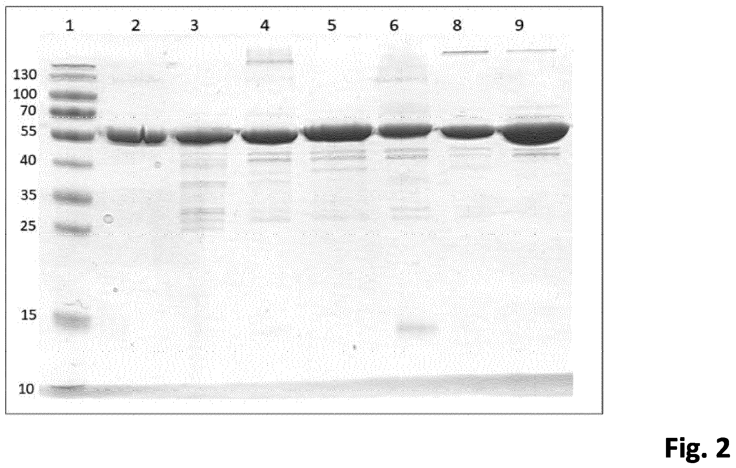

**Fig. 2** SDS-PAGE Coomassie staining of purified chimeric pyocins. Lane 1 - PageRuler Prestained protein ladder (Thermo Fisher Scientific), lane 2 - S5 (0.9 mg/ml), lane 3 - S5-PmnH (0.9 mg/ml), lane 4 - S5-Pflu095 (0.9 mg/ml), lane 5 - S5-Pflu373 (0.9 mg/ml), lane 6 - S5-Pflu794 (0.9 mg/ml), lane 7 - S5-Pflu618 (0.9 mg/ml), Lane 8 - S5-Pput259 (1.4 mg/ml). 4 µl of proteins per lane.

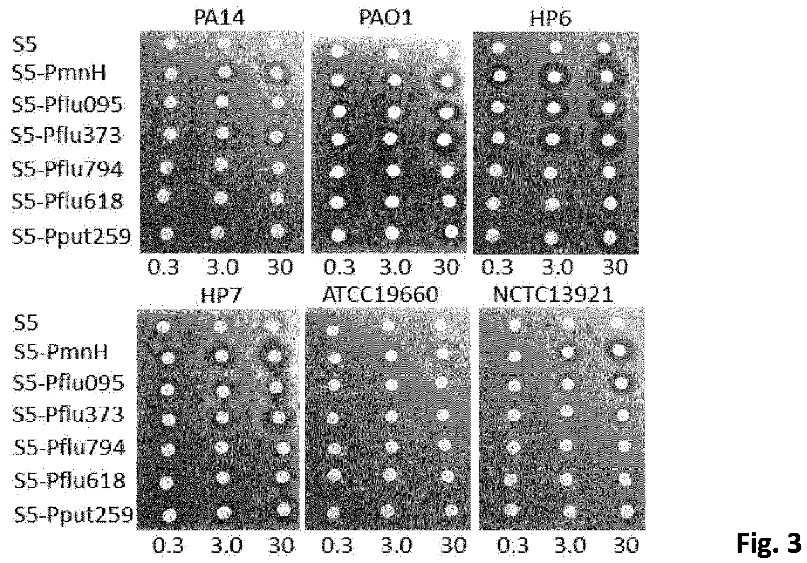

**Fig. 3** Pyocin S5 and chimeric pyocin activities on pyocin S5-producing *P*. *aeruginosa* strains. **0.3, 3 and 30 µg of S5** and chimeric bacteriocins were spotted on 6 mm Whatman discs placed on CAA agar lawns of different P. aeruginosa strains and incubated overnight.

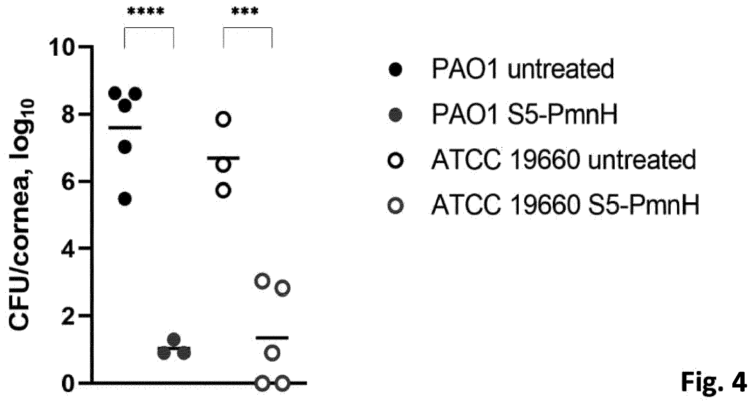

**Fig. 4** CFU counts in ex vivo porcine corneas, infected with P. aeruginosa ATCC 19660 or PAO1 and treated with S5-PmnH. 3x104 CFU of P. aeruginosa ATCC 19660 strain or 0.4x104 CFU of P. aeruginosa PAO1 strain were applied to cornea and incubated for 16-20 hours at 37 °C. Then 5 µg of S5-PmnH were applied to cornea and incubated for additional 16-20 hours. Statistical significances of the quantitative data were analyzed using GraphPad Prism software by the 1-way repeated measures ANOVA and Bonferroni's' correction for multiple comparisons. Mean is indicated by horizontal bar. ***P≤**0.001, ****P≤0.0001 vs vehicle**-treated mice.

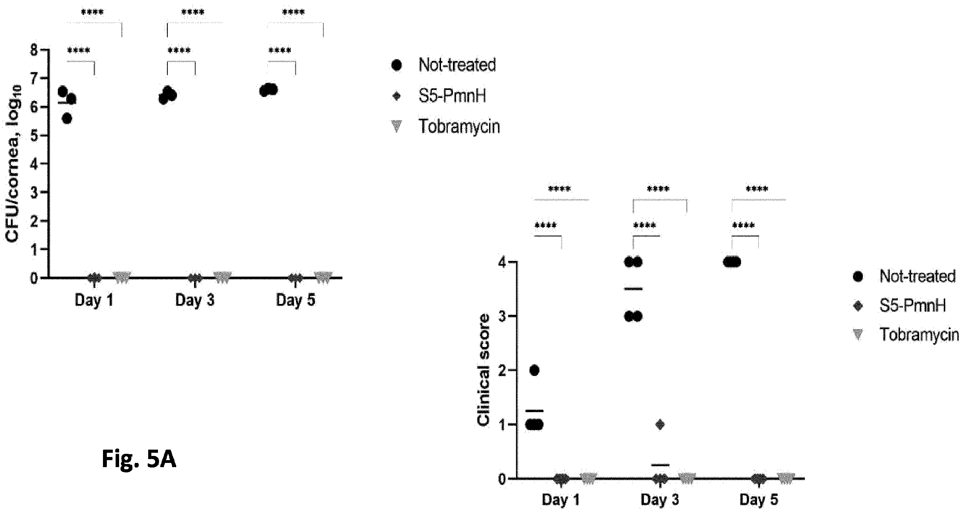

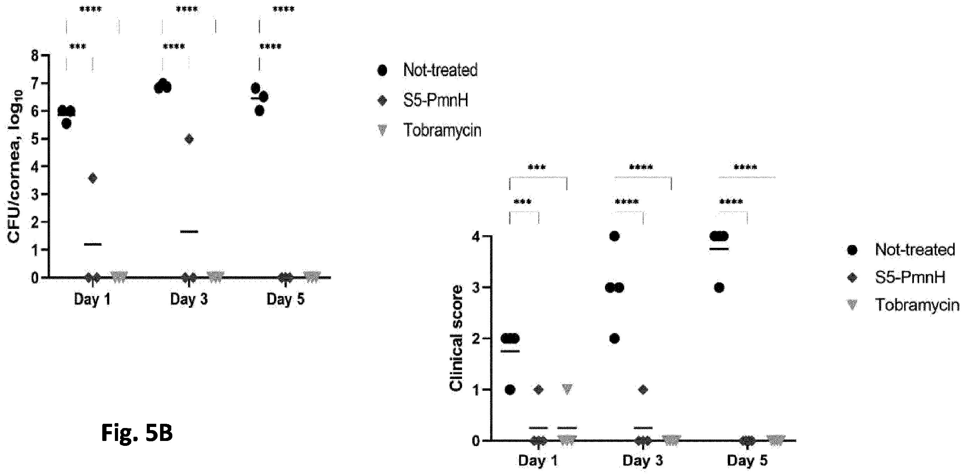

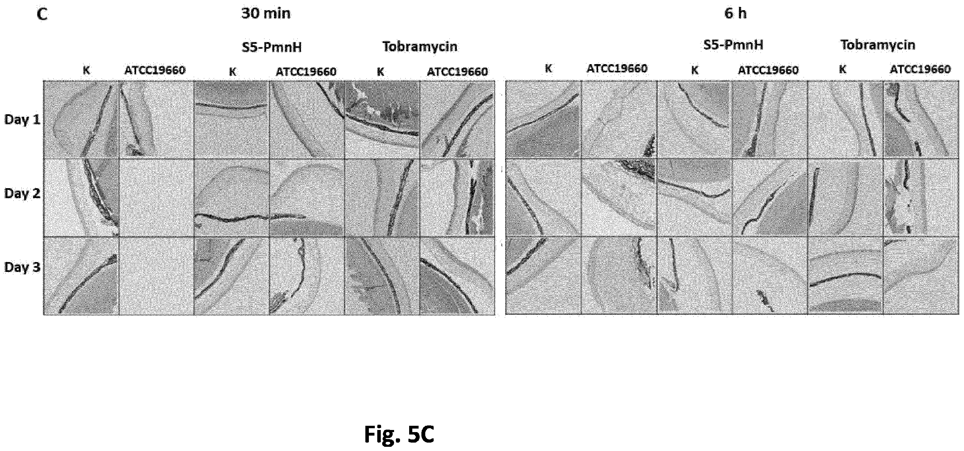

**Fig. 5** Mice cornea infection by cytotoxic strain ATCC 19660 and treatment by S5-PmnH or tobramycin. **A.** CFU counts and clinical scores of mice eyes, when treatment started 30 min post-infection. **B** - CFU counts and clinical scores of mice eyes, when treatment started 6 h post infection. **C.** Hematoxylin-eosin staining of cornea sections. **Left panel** - treatment with pyocin started 30 min post-infection. Uninfected eyes - no observed microscopic aberations. Infected control eyes: day 1 - strong corneal inflamation, days 3 and 5 - the histology was not possible because of disrupted structure of the eye. Weak edema in the corneal stroma is observed in all infected eyes treated with S5-PmnH and with tobramycin. **Right panel** - treatment with pyocin started 6 h post infection. Uninfected eyes: no marked cornea abberations. Infected untreated eyes: day 1 - thinning of corneal epithelium, thickening of stroma, acute inflammation, days 3 and 5 - acute suppurated inflammation of cornea. S5-PmnH-treated infected eyes: day 1 - acute inflammation of cornea, days 3 and 5 - no marked aberrations, week edema of corneal stroma. Tobramycin-treated infected eyes: day 3 and 5 - slight thickening of epithelium. Statistical significances of the quantitative data were analyzed using GraphPad Prism software by the 2-way repeated measures ANOVA and Dunnett's correction for multiple comparisons. Mean is indicated by horizontal bar. ***P≤0.001, ****P≤0.0001 vs vehicle-treated mice.

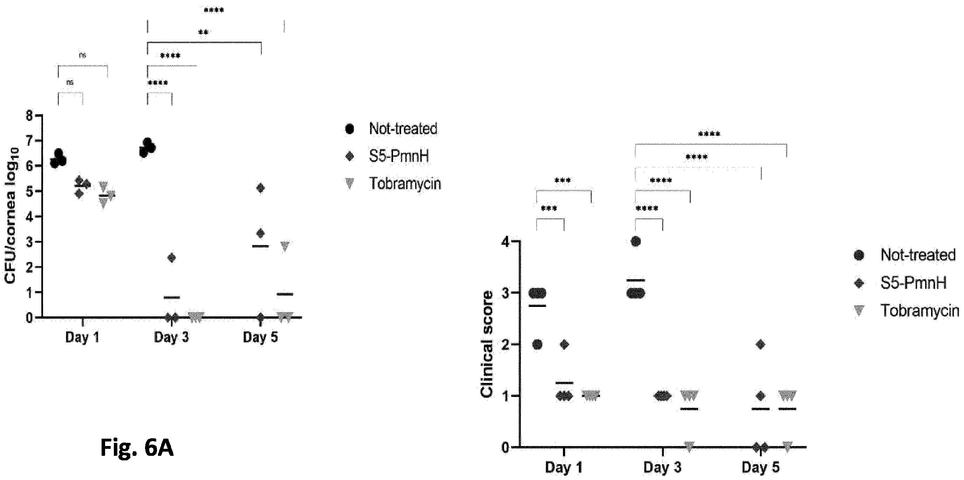

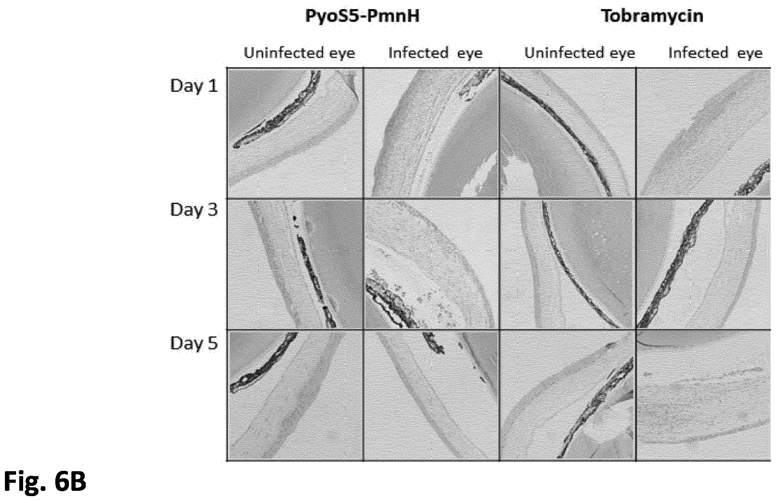

**Fig. 6** Mice cornea infection by invasive strain PAO1 and treatment by S5-PmnH or tobramycin. The corneas of left eye of mice were infected with 4×10⁶ CFU of *P*. *aeruginosa* PAO1 strain. The treatment with 20 µg of S5-PmnH or 140 µg of tobramycin started 6 h post-infection and was applied twice daily. **A**. CFU counts in mice corneas at 1, 3 and 5 dpi. **B.** Cornea clinical scores at day 1, day 3 and day 5 of experiment. **C.** Hematoxylin-eosin staining of cornea sections. Uninfected eyes: no marked aberrations, weak edema in corneal stroma observed in most samples. Infected S5-PmnH-treated eyes: days 1 and 3 - acute inflammation of cornea, day 5 - no aberrations. Infected tobramycin-treated eyes: Day 1 - acute inflammation of cornea, local cornea lesions, Day 3 - thinning of cornea epithelium, strong edema of the corneal stroma; day 5 - local inflammation of cornea, thinning and degeneration of cornea epithelium. Statistical significances of the quantitative data were analyzed using GraphPad Prism software by the 2-way repeated measures ANOVA and Dunnett's correction for multiple comparisons. Mean is indicated by horizontal bar. **P**≤0.01, ***P≤0.001, ****P≤0.0001 vs vehicle**-treated mice. Not significant (ns) P>0.05.

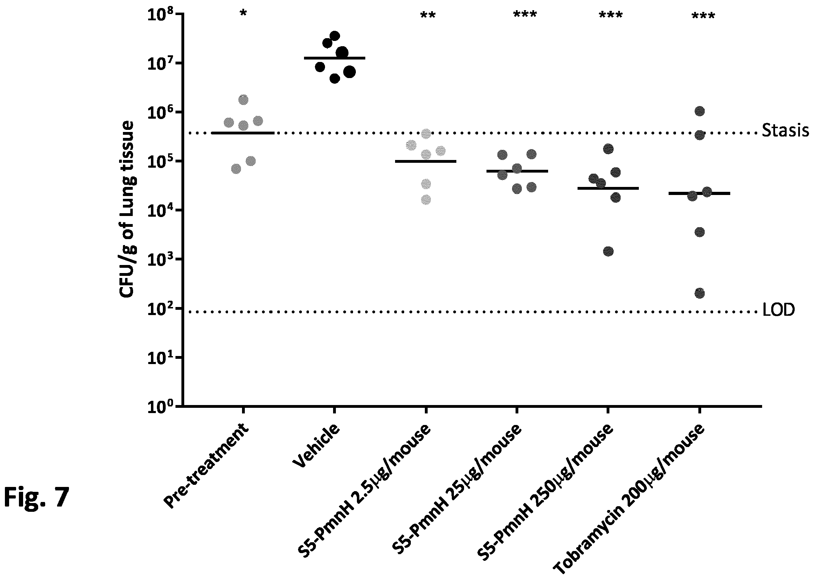

**Fig. 7****.** Scatterplot of terminal lung burden following IN infection with *P*. *aeruginosa* ATCC 27853. The data from the culture burdens were analyzed using appropriate non-parametric statistical models (Kruskal-Wallis using Conover-Inman to make all pairwise comparisons between groups) with StatsDirect (v. 3.3.3). The geometric mean burden of each treatment is indicated by the horizontal bar. *P ≤0.05, **P ≤ 0.0005, *** P ≤ 0.0001, compared to vehicle control. LOD = limit of detection.

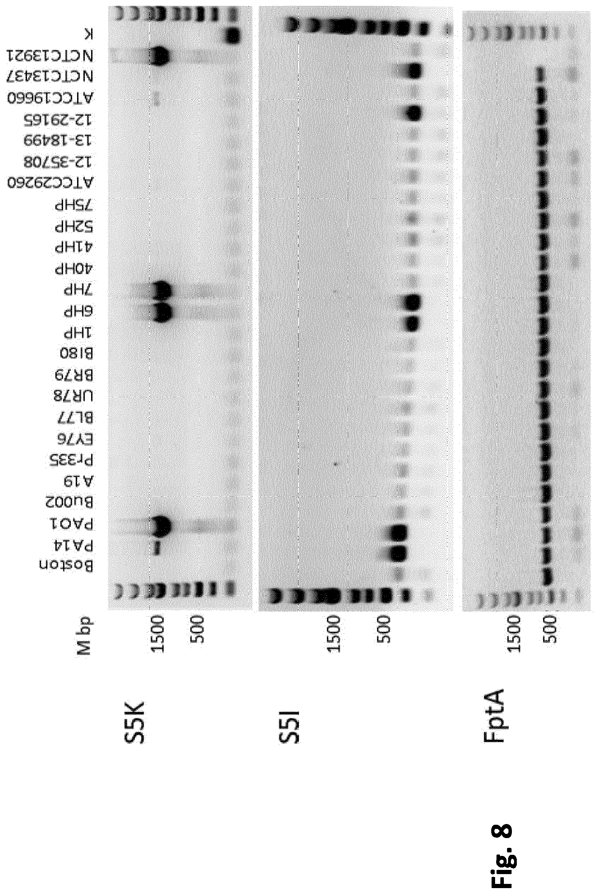

**Fig. 8**. PCR amplification of S5 killing and immunity genes and fptA from genomic DNA of 25 *P. aeruginosa* isolates. Sequence-specific primers were designed to amplify 1500 bp fragment of S5K, 330 bp fragment of S5I and 556 bp fragment of fptA. FptA is amplified in all tested strains, S5K and S5I was amplified in PA14, PAO1, HP6, HP7, ATCC 19660 and NCTC 13921 strains. K - negative control.

### DETAILED DESCRIPTION OF THE INVENTION

The bacteriocin of the invention is a colicin-type antibiotic protein. Colicins, in the case of *E. coli* colicins, are plasmid-encoded cytotoxins synthesized by *Escherichia coli,* which are secreted into the medium and kill sensitive strains of *E. coli.* Colicins may belong to different cytotoxic classes according to the mechanism by which they kill sensitive bacterial strains:
- pore-forming colicins such as ColA, CoIE1, CoIN, CoIK, Colla, Collb, and ColD, which kill cells by causing membrane depolarization
- RNase colicins,
- DNase colicins, and
- inhibitors of cell wall synthesis such as ColM.

Colicins, as well as the bacteriocins of the invention comprise, along their amino acid sequence, from the N-terminus to the C-terminus, three domains referred to as receptor binding domain, translocation domain, and killing or cytotoxicity domain (Cascales et al., Colicin Biology, Microbiology and Molecular Biology Reviews, Vol. 71, (2007) 158-229). The receptor binding domain allows binding of the colicin to a receptor on the outer membrane of a bacterial cell. The translocation domain allows binding to translocation proteins of a target bacterial cell for translocation inside the target cell. The bacteriocins of the invention are pore-forming bacteriocins. They exert cytotoxic activity by pore formation in the inner cell membrane of target cells. Thus, their target compartment of the target cells is the periplasm of the target cells. The translocation domain of the bacteriocins of the invention allows translocation of the bacteriocins into the periplasm of cells of a target pathogen.

The antibacterial protein of the invention (also referred to herein as "bacteriocin" or "pyocin") is a pore-forming bacteriocin. It comprises a polypeptide comprising a first polypeptide segment (briefly referred to herein as "first segment") and, generally contiguous thereto, a second polypeptide segment (briefly referred to herein as "second segment"). The first segment comprises the receptor binding domain and the translocation domain (from the N-terminus to the C-terminus). The second segment comprises the pore forming cytotoxic (or killing) domain. The bacteriocins of the invention are occasionally referred to herein as "chimeric" or as "chimeric pyocins", as they comprises segments that may be or are derived from different origins, notably from bacteriocins of different species of *Pseudomonas.*

Herein, a polypeptide segment (or, briefly, "segment") refers to a plurality of contiguous amino acid residues of a polypeptide or protein, the polypeptide or protein having a larger number of amino acid residues than the segment. Thus, a segment is a part of a polypeptide or protein. The term "polypeptide" refers to a polypeptide molecule (as opposed to a fragment, part or moiety of a molecule) without limitation with regard to the number of amino acid residues of the polypeptide.

The term protein covers polypeptides and combinations or complexes of two or more polypeptides, optionally with further components such as metal ions complexed by a polypeptide. Generally, a protein may be monomeric, homo- or heterooligomeric (e.g. dimeric), and may comprise further components such as metal ions complexed by one or more polypeptide.

The term "amino acid sequence" refers to the sequence of amino acid residues of a protein, polypeptide or segment; the term refers to the sequence information which is structural information of a protein, polypeptide, or segment.

The first segment of the bacteriocin of the invention may be any one selected from the following group (a-i) to (d-i):
(a-i) the segment of the amino acid sequence of SEQ ID NO: 1, or
(b-i) a segment having at least 80%, more preferably at least 85%, even more preferably at least 90%, and even more preferably at least 95% sequence identity to the amino acid sequence of SEQ ID NO: 1, or
(c-i) a segment having at least 85%, preferably at least 90%, more preferably at least 95%, and most preferably at least 98 % sequence similarity to the amino acid sequence of SEQ ID NO: 1, or
(d-i) a segment having from 1 to 60, preferably from 1 to 45, more preferably from 1 to 30, and most preferably from 1 to 15 amino acid residue substitutions, additions, insertions and/or deletions compared to the amino acid sequence of SEQ ID NO: 1.

The second segment of the bacteriocin of the invention may be any one selected from the following group (a-i)' to (d-iii)':
(a-i)' the segment of the amino acid sequence of SEQ ID NO: 3 (killing *domain of PmnH*), or
(a-ii)' the segment of the amino acid sequence of SEQ ID NO: 7 (killing *domain of Pflu095*), or
(a-iii)' the segment of the amino acid sequence of SEQ ID NO: 11 (killing *domain of Pflu373*); or
(b-i)' a segment having at least 80%, more preferably at least 85%, even more preferably at least 90%, and most preferably at least 95% sequence identity to the amino acid sequence of SEQ ID NO: 3, or
(b-ii)' a segment having at least 80%, preferably at least 85%, more preferably at least 90%, and most preferably at least 95% sequence identity to the amino acid sequence of SEQ ID NO:7, or
(b-iii)' a segment having at least 80%, preferably at least 85%, more preferably at least 90%, and most preferably at least 95% sequence identity to the amino acid sequence of SEQ ID NO:11; or
(c-i)' a segment having at least 85%, preferably at least 90%, and more preferably at least 95%, and most preferably at most 98% sequence similarity to the amino acid sequence of SEQ ID NO: 3, or
(c-ii)' a segment having at least 85%, preferably at least 90%, and more preferably at least 95%, and most preferably at most 98% sequence similarity to the amino acid sequence of SEQ ID NO: SEQ ID NO: 7, or
(c-iii)' a segment having at least 85%, preferably at least 90%, and more preferably at least 95%, and most preferably at most 98% sequence similarity to the amino acid sequence of SEQ ID NO: SEQ ID NO: 11; or
(d-i)' a segment having from 1 to 38, preferably from 1 to 28, more preferably from 1 to 19, and most preferably from 1 to 10 amino acid residue substitutions, additions, insertions and/or deletions compared to the amino acid sequence of SEQ ID NO: 3, or
(d-ii)' segment having from 1 to 38, preferably from 1 to 28, more preferably from 1 to 19, and most preferably from 1 to 10 amino acid residue substitutions, additions, insertions and/or deletions compared to the amino acid sequence SEQ ID NO: 7, or
(d-iii)' a segment having from 1 to 38, preferably from 1 to 28, more preferably from 1 to 19, and most preferably from 1 to 10 amino acid residue substitutions, additions, insertions and/or deletions compared to the amino acid sequence SEQ ID NO: 11.

The wording "the segment of the amino acid sequence of SEQ ID NO:z", z meaning any one of the SEQ ID NOs referred to, means that the amino acid sequence of the segment is that of SEQ ID NO:z, i.e. the entire amino acid sequence of SEQ ID NO:z. The wording "a segment having at least ... sequence identity/similarity to the amino acid sequence of SEQ ID NO: z" means that the amino acid sequence of the segment has at least the indicated amino acid sequence identity or similarity, respectively, to the entire amino acid sequence of SEQ ID NO: z, and preferably has at least the number of amino acid residues as the SEQ ID NO referred to. The SEQ ID NOs with respect to which segments or amino acid sequences are defined are generally referred to herein as reference sequences.

Herein, the determination of sequence identities and similarities is done using Align Sequences Protein BLAST (BLASTP 2.6.1+) (Stephen F. Altschul, Thomas L. Madden, Alejandro A. Schaffer, Jinghui Zhang, Zheng Zhang, Webb Miller, and David J. Lipman (1997), "Gapped BLAST and PSI-BLAST: a new generation of protein database search programs", Nucleic Acids Res. 25:3389-3402.).

Where a polypeptide or segment is defined herein by a number or number range of amino acid residue substitutions, additions, insertions and/or deletions, amino acid residue substitutions, additions, insertions or deletions may be combined, but the given number or number or number range refers to the sum of all amino acid residue substitutions, additions, insertions and deletions. Among amino acid residue substitutions, additions, insertions and deletions, amino acid substitutions, additions, and deletions are preferred. The term "insertions" relates to insertions of amino acid residues within the amino acid sequence of a reference sequence, i.e. excluding additions at the C- or N-terminal end of the reference sequence. The term additions means additions of amino acid residues at the C- or N-terminal end of the amino acid sequence of a reference sequence. A deletion may be a deletion of a terminal or an internal amino acid residue of a reference sequence. Herein, where a polypeptide or segment is defined by a number or number range of amino acid residue substitutions, additions, insertions and/or deletions relative to a reference sequence, in a further embodiment, the polypeptide or segment may have from 1 to several amino acid residue substitutions, additions, insertions or deletions relative to the indicated amino acid sequence of segment.

In the bacteriocin of the invention, the first segment is preferably N-terminal to said second segment in the amino acid sequence of said bacteriocin.

Herein, in any item (x-y)' (wherein x stands for any one of a, b, c, or d, and y stands for any roman numeral i to iii), the prime ' indicates killing domains or segments. Items (x-y) lacking the prime indicate the segments comprising the receptor binding and translocation domains. Among items (a) to (d), those of items (a), (b) and (d) are preferred and items (a) and (d) are more preferred. Similarly, among items (a)' to (d)', those of items (a)', (b)' and (d)' are preferred and items (a)' and (d)' are more preferred. This applies analogously to combinations of items (a) to (d) and (a)' to (d)', where combinations of the same letter a to d are preferred.

Where the protein of the invention comprises a first segment as defined above and a second segment as defined above, any first segment as defined above may be combined with any second segment. In one embodiment, a binding and translocation segment of any item (a) to (d) is combined with a catalytic domain or segment of any item (a)' to (d)', respectively.

In a first general embodiment, a first segment of any one of items (a) to (d) is combined with a second segment according to any one of items (x-i)', i.e. with a killing domain of PmnH or a derivative thereof as defined in any one of items (x-i)'.

In a second general embodiment, a first segment of any one of items (a) to (d) is combined with a second segment according to any one of items (x-ii)', i.e. with a killing domain of Pflu095 or a derivative thereof as defined in any one of items (x-ii)'.

In a third general embodiment, a first segment of any one of items (a) to (d) is combined with a second segment according to any one of items (x-iii)', i.e. with a killing domain of Pflu373or a derivative thereof as defined in any one of items (x-iii)'.

The bacteriocin preferably has a minimum inhibitory concentration (MIC) as determined by the assay of Example 2 against *P. aeruginosa* PAO1 of at most 10 times, more preferably at most 5-times, even more preferably at most 2.5 times of that of S5-Pflu095 of SEQ ID NO: 5.

The first and second segment may be or may not be linked by a peptide linker. Preferably, no peptide linker is present. If a peptide linker is used, the peptide linker may consist of from 1 to 50 amino acid residues, preferably from 1 to 30, and more preferably from 1 to 10 amino acid residues. A peptide linker may be selected so as not to increase its MIC in the assay of Example 2 against *P. aeruginosa* PAO1, compared to a bacteriocin lacking the linker but having otherwise the same amino acid sequence, by more than 5-fold, preferably not more than 2- fold. Alternatively, the condition of the previous paragraph may be applied.

The bacteriocin of the invention may alternatively be defined as comprising a polypeptide that comprises or consists of:
(A-i) the amino acid sequence of SEQ ID NO: 5 (sequence of protein S5-PmnH), or
(A-ii) the amino acid sequence of SEQ ID NO: 9 (sequence of protein S5-Pflu095), or
(A-iii) the amino acid sequence of SEQ ID NO: 13 (sequence of protein S5-Pflu373); or
(B-i) an amino acid sequence having at least 80%, more preferably at least 85%, even more preferably at least 90%, and even more preferably at least 95% sequence identity to the amino acid sequence of SEQ ID NO: 5, or
(B-ii) an amino acid sequence having at least 80%, more preferably at least 85%, even more preferably at least 90%, and even more preferably at least 95% sequence identity to the amino acid sequence of SEQ ID NO: 9, or
(B-iii) an amino acid sequence having at least 80%, more preferably at least 85%, even more preferably at least 90%, and even more preferably at least 95% sequence identity to the amino acid sequence of SEQ ID NO: 13; or
(C-i) an amino acid sequence having at least 85%, preferably at least 90%, and more preferably at least 95%, and most preferably at least 98% sequence similarity to the amino acid sequence of SEQ ID NO: 5, or
(C-ii) an amino acid sequence having at least 85%, preferably at least 90%, and more preferably at least 95%, and most preferably at least 98% sequence similarity to the amino acid sequence of SEQ ID NO: 9, or
(C-iii) an amino acid sequence having at least 85%, preferably at least 90%, and more preferably at least 95%, and most preferably at least 98% sequence similarity to the amino acid sequence of SEQ ID NO: 13; or
(D-i) an amino acid sequence having from 1 to 100, preferably from 1 to 75, more preferably from 1 to 50, even more preferably from 1 to 25, and most preferably from 1 to 12 amino acid residue substitutions, additions, insertions and/or deletions to the amino acid sequence of SEQ ID NO: 5, or
(D-ii) an amino acid sequence having from 1 to 100, preferably from 1 to 75, more preferably from 1 to 50, even more preferably from 1 to 25, and most preferably from 1 to 12 amino acid residue substitutions, additions, insertions and/or deletions to the amino acid sequence of SEQ ID NO: 9; or
(D-iii) an amino acid sequence having from 1 to 100, preferably from 1 to 75, more preferably from 1 to 50, even more preferably from 1 to 25, and most preferably from 1 to 12 amino acid residue substitutions, additions, insertions and/or deletions to the amino acid sequence of SEQ ID NO: 13.

The wording "the amino acid sequence of SEQ ID NO: z" in items (A-y) means that the amino acid sequence of the polypeptide is or comprises that of the reference sequence (SEQ ID NO: z). The wording "an amino acid sequence having at least ... sequence identity/similarity to the amino acid sequence of SEQ ID NO: z" of items (B-y) or (C-y), respectively, means that the amino acid sequence of the polypeptide has at least the indicated amino acid sequence identity or similarity, respectively, to the entire amino acid sequence of SEQ ID NO: z, and preferably has at least the number of amino acid residues as the SEQ ID NO referred to. Herein, the determination of sequence identities and similarities is done as defined above.

The wording "an amino acid sequence having from ... amino acid residue substitutions, additions, insertions and/or deletions to the amino acid sequence of SEQ ID NO: z" (SEQ ID NO: z stands for the reference sequence) of items (D-y) means that the amino acid sequence of the polypeptide has the indicated number or number range of amino acid residue substitutions, additions, insertions and/or deletions to the entire amino acid sequence of the reference sequence. Otherwise, the definitions and preferred embodiments defined above apply analogously.

The bacteriocin of the invention may, in addition to the definition given above, comprise a further segment at the N- and/or C-terminus, such as a purification tag, signal sequence, etc. In one embodiment, however, the polypeptide of the bacteriocin contains no further segment, but the polypeptide of the bacteriocin consists of the definitions above.

The bacteriocin preferably has a minimum inhibitory concentration (MIC) as determined by the assay of Example 2 against *P. aeruginosa* PAO1 of at most 10 times, more preferably at most 5-times, even more preferably at most 2.5 times of that of S5-Pflu095 of SEQ ID NO: 5.

The invention also provides a nucleic acid molecule and a nucleic acid construct, comprising a nucleic acid sequence encoding a bacteriocin according to the invention. The acid molecule and nucleic acid construct may comprise a transcription promoter that is preferably active in the cells wherein the bacteriocin is to be expressed, such as bacterial or plant cells.

The invention also provides a bacterial or eukaryotic cell, comprising the nucleic acid molecule according to the invention. The eukaryotic cell may be a mammalian cell or a plant cell. The plant cell may be a cell of a plant wherein the bacteriocin is or is to be expressed. Further, a plant comprising the nucleic acid molecule according to the invention is provided. The plant may be any one of those mentioned below.

The invention further provides a composition comprising a (first) bacteriocin of the invention and optionally further components as the case requires such as one or more carriers or excipients. The composition may further comprise a second or further bacteriocin of the invention. Second bacteriocin means that it comprises a polypeptide, the amino acid sequence of which differs from that of the first bacteriocin. Preferably, the second bacteriocin has a killing domain from another origin than the first bacteriocin. Among the bacteriocins of items (X-i) to (X-iii), X being selected from A, B, C, D, this means that a bacteriocin of item (X-i) may, for example, be combined with a bacteriocin of item (X-ii) or (X-iii).

As the bacteriocin of the invention may be produced by expression in plants or cells thereof, the composition may be a plant material or extract of the plant material, wherein the plant material may be a material from a plant having expressed the bacteriocin, preferably *Nicotiana* or an edible plant having expressed said bacteriocin. An extract of the plant material may be an aqueous solution containing the bacteriocin of the invention that is present or expressed in said plant material and optionally other water-soluble components, or a dried product of such aqueous solution. The extract preferably has water-insoluble components of the plant material removed e.g. by filtration or centrifugation. The plant material may be a material from a plant selected from the group consisting of spinach, chard, beetroot, carrot, sugar beet, leafy beet, amaranth, *Nicotiana,* and/or said plant material may be one or more leaves, roots, tubers, or seeds, or a crushed, milled or comminuted product of said leaves, roots, tubers, or seeds. In a preferred embodiment, notably for use as a pharmaceutical composition, the bacteriocin is purified to a level required by the respective regulatory provisions for the intended application or use of the bacteriocin or composition of the invention.

The composition (such as said extract from a plant) may be a solid or liquid composition, such as a solution or a dispersion, containing the bacteriocin(s) of the invention. The liquid composition may be aqueous, such as an aqueous solution. The concentration of the (one or more) bacteriocin(s) in said aqueous dispersion or solution may be from 0.0001 to 10 mg/ml, preferably from 0.001 to 5.0 mg/ml, more preferably from 0.005 to 1.0 mg/ml, and most preferably from 0.01 to 0.2 mg/ml. If more than one bacteriocin capable of exerting a cytotoxic effect on *Pseudomonas* is employed, these concentrations relate to the total concentration of all such bacteriocins of the invention.

The aqueous solution as said composition may, apart from the one or more bacteriocin, contain a buffer. The buffer may be an inorganic or organic acid and/or salts thereof. An example of an inorganic acid is phosphoric acid or salts thereof. Examples of the organic acid are HEPES, acetic acid, succinic acid, tartaric acid, malic acid, benzoic acid, cinnamic acid, glycolic acid, lactic acid, citric acid, and ascorbic acid. Preferred organic acids are malic acid, lactic acid, citric acid, and ascorbic acid. The pH of the solution may generally be from 6 to 8, preferably from 6.5 to 7.5. The pH of the solution containing the buffer may be adjusted using hydrochloric acid or sodium hydroxide solution.

Further, the solution may contain isotonic agents such as glycerol, mannitol, sorbitol, a salt, or a combination of two or more such compounds. The salt is preferably an alkali metal salt. A preferred salt to be used is sodium chloride. The aqueous solution containing the one or more bacteriocin may be a buffered aqueous solution that may contain further solutes e.g. salts such as from 50 to 400 mM NaCl, preferably from 100 to 200 mM NaCl. The aqueous solution may further contain a sulfhydryl compound such as dithiothreitol (DTT), dithioerythritol, thioethanol or glutathione, preferably DTT. The concentration of the total of sulfhydryl compounds in the aqueous solution may be from 1 to 50 mM, preferably from 2 to 20 mM and more preferably from 4 to 10 mM.

Examples of carriers of the composition are solvents such as water or an aqueous buffer (as described above); salts; sugars such as monosaccharides and disaccharides; sugar alcohols; hydrophilic polymers such as polysaccharides, polyethylene glycol, or hyaluronic acid (or its sodium salt); and other carriers known from pharmaceutical compositions. Examples of polysaccharides are starch, cellulose and cellulose derivatives such as hydroxypropyl cellulose, hydroxypropyl methylcellulose (HPMC), and carmellose. Examples of sugars are glucose, fructose, lactose, sucrose, and maltose.

If the composition of the invention is a solid composition, it may be a powder such as a lyophilized solid composition obtained by lyophilization of the extract or solution mentioned above. The powder may contain additional solid components such as those mentioned above for the aqueous solution. Before use, it may be reconstituted with a suitable liquid, such as water or buffer. The solid composition may contain buffer, salts or other components as mentioned above, such that the concentrations given above may be achieved upon reconstitution or dissolution of the solid composition.

In a preferred embodiment, the composition is a pharmaceutical composition that comprises one or more pharmaceutically acceptable carrier(s) or excipient(s). Examples of possible carrier(s) and excipients were given above. A preferred pharmaceutical composition is a liquid aqueous solution of the one or more bacteriocins. In another embodiment, the composition is a solid formulation suitable for administration as an aerosol to a patient.

For the treatment of keratitis, the pharmaceutical composition may be an ophthalmic composition comprising a bacteriocin according to the invention. The ophthalmic composition may be a sterile buffered solution of pH 7.0 to 7.5 having an osmolarity of 250-350 mOsm/L, preferably 270-330 mOsm/L. Possible buffers, if used, for ophthalmic compositions are phosphate, citrate, tris (trometamol), or sodium hydrogencarbonate; citrate is preferred. In one embodiment, the buffer is phosphate-free, and phosphate-free citrate-buffered ophthalmic compositions are preferred. The concentration of the one or more bacteriocin may be from 0.1 to 10 mg/mL, preferably from 0.5 to 8 mg/mL, more preferably from 1.0 to 5 mg/mL. As a wetting agent, a polysaccharide as mentioned above or sodium hyaluronate may be contained as well.

The bacteriocin and the composition of the invention may be used in therapy. They may be used in the treatment or prevention of a bacterial infection, preferably of a bacterial infection by *Pseudomonas,* such as *P. aeruginosa.* In one embodiment, the bacteriocin or the composition is used in the treatment of bacterial lung infection or bacterial pneumonia, preferably of lung infection or pneumonia caused by *Pseudomonas,* such as *P. aeruginosa.* In another embodiment, the bacteriocin or the composition is used in the treatment of keratitis, preferably of bacterial keratitis caused by *Pseudomonas,* such as *P. aeruginosa.*

The invention also provides a method of treating infection with *Pseudomonas,* such as *P. aeruginosa* of a subject in need thereof, comprising administering to said subject one or more bacteriocin as described above or a composition as described above. The administration is preferably topical, e.g. to the eyes in the case of keratitis or to the lungs in the case of lung infection. The subject may be a human being or a mammal such as a farm animal. Humans are preferred subjects. Generally, a liquid or solid pharmaceutical composition containing the bacteriocin(s) and optionally further components as described above is prepared for administration to the mammal. Liquid compositions may be aqueous solutions as described above. Solid compositions may be powder containing the at least one bacteriocin(s) e.g. in freeze-dried form.

For the treatment of keratitis, the bacteriocin(s) or the pharmaceutical composition is administered into an affected eye, preferably in the form of an ophthalmic composition as described above. The solution may be dropwise instilled into an affected eye. The solution may be administered one drop into an affected eye three times per day. The duration of treatment may be from one day until the infection has been effectively treated, which may be from 2 to 10 days.

For the treatment of lung infection, the bacteriocin or the pharmaceutical composition is administered into the lungs of the subject in need thereof. The bacteriocin or the pharmaceutical composition may be administered into the lungs as a solid or liquid aerosol. Thus, the invention also provides a pulmonary formulation comprising the bacteriocin of the invention or the composition according to the invention. For an overview over topical lung delivery of protein therapeutics see e.g. Bodier-Montagutelli et al., EXPERT OPINION ON DRUG DELIVERY 2018, VOL. 15, NO. 8, 729-736; doi.org/10.1080/17425247.2018.1503251. The formulation may be a dry powder for aerosolization or a liquid solution for nebulization. The composition may be administered to a subject 1 to 3 times per day. The duration of treatment may be from one day until the infection has been effectively treated, which may be from 2 to 10 days. The dosage may be from 10 µg to 500 µg, preferably from 50 to 200 µg of bacteriocin of the invention per administration for a human subject.

A bacteriocin according to the invention may be produced by known methods of protein expression in a standard expression system. Co-expression of the immunity protein of the bacteriocin may be beneficial for preventing a toxic effect of the bacteriocin on the host organism. For producing the bacteriocin, a nucleotide sequence encoding it may be expressed in a suitable host organism. Methods usable for producing and purifying a protein of interest have been described in the prior art and any such methods may be used. An *E. coli* expression system as generally known in the art may, for example, be used. If a eukaryotic expression system is used, one or more introns may be inserted in the coding sequence of the bacteriocin to prevent toxicity on the bacterial organism used for cloning.

Particularly efficient expression methods are plant expression systems that are also known in the prior art. Plant expression systems usable for expressing a bacteriocin according to the invention are described in the Examples. A possible way of achieving expression of a nucleotide sequence of interest in plants is the use of self-replicating (viral) replicons containing the nucleotide sequence encoding the bacteriocin. The coding sequence of the bacteriocin may be codon optimized for expression in plants or in the particular plant used as expression host. Plant viral expression systems have been described in many publications, such as in WO2012019660, WO2008028661, WO2006003018, WO2005071090, WO2005049839, WO2006012906, WO02101006, WO2007137788 or WO02068664 and many more publications are cited in these documents. Various methods for introducing a nucleic acid molecule, such as a DNA molecule, into a plant or plant part for transient expression are known. Agrobacteria may be used for transfecting plants with the nucleic acid molecule (vector) or nucleic acid construct e.g. by agroinfiltration or spraying with agrobacterial suspensions. For references, see WO 2012019660, WO 2014187571, or WO 2013149726.

In embodiments wherein strong expression of a bacteriocin as a protein of interest is desired, a nucleic acid construct containing a nucleotide sequence encoding the bacteriocin may encode a viral vector that can replicate in plant cells to form replicons of the viral vector. In order to be replicating, the viral vector and the replicons may contain an origin of replication that can be recognized by a nucleic acid polymerase present in plant cells, such as by the viral polymerase expressed from the replicon. In case of RNA viral vectors (referred to as "RNA replicons"), the replicons may be formed by transcription under the control of a promoter active in plant cells, from the DNA construct after the latter has been introduced into plant cell nuclei. In case of DNA replicons, the replicons may be formed by recombination between two recombination sites flanking the sequence encoding the viral replicon in the DNA construct, e.g. as described in WO00/17365 and WO 99/22003. If the replicon is encoded by the DNA construct, RNA replicons are preferred. Use of DNA and RNA viral vectors (DNA or RNA replicons) has been extensively described in the literature over the years. Some examples are the following patent publications: WO2008028661, WO2007137788, WO2006003018, WO2005071090, WO2005049839, WO02097080, WO02088369, WO02068664. Examples of DNA viral vectors are those based on geminiviruses. For the present invention, viral vectors or replicons based on plant RNA viruses, notably those based on plus-sense single-stranded RNA viruses may be preferably used. Accordingly, the viral replicon may be a plus-sense single-stranded RNA replicon. Examples of such viral vectors are those based on tobacco mosaic virus (TMV) and potexvirus X (PVX). "Based on" means that the viral vector uses the replication system such as the replicase and/or other proteins involved in replication of these viruses. Potexvirus-based viral vectors and expression systems are described in EP2061890 or WO2008/028661. The method described in reference 11 may be used.

The bacteriocin may be expressed in a multi-cellular plant or a part thereof, notably a higher plant or parts thereof. Both monocot and dicot (crop) plants can be used. Common plants usable for expressing the protein of interest include *Nicotiana benthamiana, Nicotiana tabacum,* spinach, *Brassica campestris, B. juncea,* beets *(Beta vulgaris),* cress, arugula, mustard, strawberry, *Chenopodium capitatum,* lettuce, sunflower, cucumber, chinese cabbage, cabbage, carrot, green onion, onion, radish, lettuce, field peas, cauliflower, broccoli, burdock, turnip, tomato, eggplant, squash, watermelon, prince melon, and melon. Preferred plants are spinach, chard, beetroot, carrot, sugar beet, *Nicotiana tabacum,* and *Nicotiana benthamiana.* In one embodiment, plants are used that do not normally enter the human or animal food chain such as *Nicotiana* species such as *N. tabacum* and *N. benthamiana.*

Generally, the bacteriocin as a protein of interest is expressed in the cytosol of cells of the plants or plant parts. In this case, no signal peptide directing the protein of interest into a particular compartment is added to the protein. Alternatively, the protein of interest can be expressed in or targeted into chloroplasts of the plants; in the latter case, an N-terminal pre-sequence, generally referred to as plastid transit peptide or chloroplast targeting peptide, is added to the N-terminal or C-terminal end, preferably the N-terminal end, of the bacteriocin as the protein of interest. The bacteriocin may be co-expressed together with an immunity protein.

In the process of producing a composition comprising at least one bacteriocin, a bacteriocin may, in the first step, be expressed in a plant or cells of a plant, such as an edible plant. In the next step, plant material containing expressed bacteriocin from a plant having expressed the bacteriocin is harvested. Plant material may e.g. be leaves, roots, tubers, or seeds, or a crushed, milled or comminuted product of leaves, roots, tubers, or seeds. In step (iii), the bacteriocin is extracted from the plant material using an aqueous buffer. This may include that the plant material is homogenized and insoluble material may be removed by centrifugation or filtration. Soluble components including the bacteriocin will be extracted into the aqueous buffer to produce a bacteriocin solution in the aqueous buffer. The aqueous buffer may contain an inorganic or organic acid or salts thereof and may have a pH as defined above for the aqueous solution as a composition of the invention. Further, the aqueous buffer may contain salt and/or a sulfhydryl compound as also described above for the aqueous solution as a composition of the invention. If a relatively pure bacteriocin composition is desired, the bacteriocin solution in the aqueous buffer may be further purified by removing undesired components in step (iv) according to known methods of protein purification. The bacteriocin may be purified as described in reference¹¹.

### EXAMPLES

### Reference Example 1: Bacterial strains and cultures

Unless otherwise stated, *P. aeruginosa* strains were prepared by culturing in Lysogeny Broth (LB) medium (Roth) or Casamino Acids (0.5 % Bacto^{™} Casamino acids, 5.2 mM K₂HPO₄, 5 mM MgSO₄) medium (BD Bacto) at 37 °C under shaking conditions (200 rpm); overnight cultures were prepared by inoculation from frozen stocks. *P. aeruginosa* strains used in experiments are described in **Suppl. Table 1**.

**Suppl. Table 1. P. aeruginosa strains used in the invention.**

| ***P. aeruginosa* strain** | **Isolated from** | **Source** |
|---|---|---|
| Boston 41501 (ATCC 27853) | Blood culture | LGC ATCC |
| PAO1 (ATCC 15692, DSM 22644) | Infected wound | Leibniz Institut DSMZ-Deutsche Sammlung von Mikroorganismen und Zellkulturen GmbH |
| PA14 (DSM19882) | | Leibniz Institut DSMZ-Deutsche Sammlung von Mikroorganismen und Zellkulturen GmbH |
| Bu002 LMG24892 | Infected wound | The BCCM/LMG bacteria collection |
| A19 LMG25088 | Infected wound | The BCCM/LMG bacteria collection |
| Pr335 LMG24969 | Hospital environment | The BCCM/LMG bacteria collection |
| EY76 | Infected eye | **A. Vitkauskienė LSMU** |
| BL77 | Blood | **A. Vitkauskienė LSMU** |
| UR78 | Urinary tract infection | **A. Vitkauskienė LSMU** |
| BR79 | Bronchus | A. **Vitkauskienė LSMU** |
| BI80 | Biopsy | **A. Vitkauskienė LSMU** |
| HP1 | Hospital pneumonia | A. **Vitkauskienė LSMU** |
| HP6 | Hospital pneumonia | A. Vi**tkauskienė LSMU** |
| HP7 | Hospital pneumonia | **A. Vitkauskienė LSMU** |
| HP40 | Hospital pneumonia | **A. Vitkauskienė LSMU** |
| HP41 | Hospital pneumonia | **A. Vitkauskienė LSMU** |
| HP52 | Hospital pneumonia | A. **Vitkauskienė LSMU** |
| HP75 | Hospital pneumonia | **A. Vitkauskienė LSMU** |
| PA-103 | Sputum of patient | ATCC 29260 |
| 12-35708 | Cystic fibrosis | **A. Vitkauskienė** LSMU |
| 13-18499 | Cystic fibrosis | **A. Vitkauskienė LSMU** |
| 12-29165 | Cystic fibrosis | **A. Vitkauskienė LSMU** |
| 180 (ATCC 19660) | Human septicemia | LGC ATCC |
| NCTC 13437 | | Public Health England Culture collections |
| NCTC 13921 | Human throat | Public Health England Culture collections |

### Example 1: Construction of chimeric pyocins

The open reading frames encoding for PmnH (*P. synxantha* EIK72868), Pflu095 (*P. fluorescens* WP_016979095), Pflu373 (*P. fluorescens* WP_014717373), Pflu794 (*P. fluorescens* WP_081041794), Pflu618 (*P. fluorescens* WP_034155618) and Pput259 (*P. putida* WP_098964259) optimized for expression in *N. benthamiana* were synthetized by Thermofisher Scientific (USA). Pyocin S5 synthesis and expression vector construction were described previously¹¹.

Chimeric proteins were constructed as follows: N-terminal end of pyocin S5 (coding sequence of 1-310 aa containing receptor binding and translocation domains of this pyocin) was amplified with sequence-specific primers flanked by *Bsal* recognition sites **(Suppl. Table 2).** Cytotoxic domains of all non-*P*. *aeruginosa* putative bacteriocins were amplified with sequence specific primers flanked with Bsal recognition sites **(Suppl. Table 2).** Each killing domain fragment was paired with S5 fragment and both fragments were inserted in Bs*al* digested pICH29912, assembled TMV-based MagnICON vector³⁹. Obtained plasmids were used to transform *A. tumefaciens* GV3101. The sequences of chimeric pyocins are presented below.

**Suppl Table 2.** Primers used to amplify receptor-binding- and translocation domain of S5 and pore-forming domains of PmnH, Pflu095, Pflu794, Pflu618 and Pput259. In *italics* - Bsal site, underlined - overlapping sequence obtained after amplification, **bold** - sequence complementary to template (synthetic gene with plant-optimized codons).

| **SEQ ID NO:** | **Primer** | **Sequence** |
|---|---|---|
| 27 | S5 fwd | AAA***GGTCTC*ACATGTCCAATGATAAC** |
| 28 | S5 rev | AAA*GGTCTC*A**TTCTGTTCTCCTTCTTGTAGTCT** |
| 29 | PmnH fwd | AAA*GGTCTC*AAGAA**AAGGAACTTACACCAGATGAGAA** |
| 30 | PmnH rev | AAA*GGTCTC***AAAGCTTAATTCAAAATAAG** |
| 31 | Pflu095 fwd | AAA*GGTCTC*AAGAA**AAGGATAAGGTTAGGACTGAGG** |
| 32 | Pflu095 rev | AAA*GGTCTC*A**AAGCTTAAAGGCTGG** |
| 33 | Pflu794 fwd | AAA*GGTCTC*AAGAA**AAGGCTAGCGACCAGGCTAAC** |
| 34 | Pflu794 rev | AAA*GGTCTC*A**AAGCTTAGAAGCCGA** |
| 35 | Pflu618 fwd | AAA*GGTCTC*AAGAA**GCTGAAGCTGAGGCTAAGAGA** |
| 36 | Pflu618 rev | AAA*GGTCTC*A**AAGCTTACCTTTGCTC** |
| 37 | Pput259 fwd | AAA*GGTCTC*AAGAA**GCAGAGGCTGAGGCTAAGAG** |
| 38 | Pput259 rev | AAA*GGTCTC*A**AAGCTTAGTGAGCAG** |

### Chimeric pyocin expression in plants

*N. benthamiana* plants were grown in a growth chamber at 25 °C with a 16 h light and 8 h dark photoperiod. Four-to-six-week-old plants were used for vacuum infiltration with recombinant *A. tumefaciens.*

*Agrobacterium* strains were inoculated from frozen stocks in 4 ml LB medium containing 50 µg/ml rifampicin and 50 µg/ml kanamycin and cultivated at 28 °C with shaking at 220 rpm. Overnight cultures were diluted 1:1000 starting from OD₅₉₅=1.0 in tap water and supplemented with 0.05 % Silwet L77 (Kurt Obermeier). *Agrobacterium* suspension was poured into a desiccator vessel, connected to a vacuum pump. The entire leaf system of a plant was then submerged into the suspension. Agroinfiltration was achieved by applying (till pressure of 200 mbar) and releasing vacuum through the pump. Plant leaves were harvested 5-6 days post agroinfiltration.

### Purification of plant-produced chimeric pyocins

Frozen leaf tissue was homogenized with chilled mortar and pestle in liquid nitrogen. Prepared powder was mixed with cold extraction buffer (50 mM Tris, 5 mM sodium acetate, pH 5.0) at a ratio of 1 g of plant material to 5 ml of buffer. The crude extract was incubated at 20-25 °C for 15-20 min. Cell debris were removed by centrifugation at 3220 g, at 4 °C for 20 min. Pellets were discarded and the supernatant was filtered through membrane filter (pore size 0.45 µm). Ammonium sulphate was added up to 0.60 M and pH of solution adjusted to 8.0. The formed precipitate was removed by centrifugation at 3220 g, at 4 °C for 5 min. The supernatant was taken as total soluble protein and applied for purification in two steps.

At the first purification step, the chromatography column was filled with Phenyl Sepharose FF resin (GE Healthcare Life Sciences, Uppsala, Sweden) and pre-equilibrated with cold buffer (50 mM Tris, 5 mM sodium acetate, 0.60 M (NH₄)₂SO₄, pH 8.0). Protein solution was loaded to column and the Phenyl Sepharose bounded protein fraction was eluted by washing with elution buffer (50 mM Tris, 5 mM sodium acetate, 0.30 M (NH₄)₂SO₄, pH 8.0). Collected protein fraction was loaded into the diafiltrating concentrator (10 kDa) and centrifuged at 3220 g until the volume of protein solution decreased 10 fold. The concentrate was then diluted up to a primary volume with 50 mM Tris and 5 mM sodium acetate (pH 8.0). The procedure was repeated till conductivity decreased below 5 mS/cm and afterwards the protein solution was subjected to the final purification step using Q Sepharose FF resin (GEHealthcare Life Sciences, Uppsala, Sweden). Chromatography media was pre-equilibrated with cold buffer (50 mM Tris, 5 mM Sodium acetate, pH 8.0). Protein solution was loaded to column and Q Sepharose unbounded protein was collected in flow-through fraction. Collected protein fraction was loaded into the diafiltrating concentrator (10 kDa) and centrifuged at 3220 g until the volume of protein solution decreased 10 folds. The concentrate was diluted up to a primary volume with phosphate-buffered saline (PBS) buffer. The protein was freeze-dried for long time storage. Pyocin S5 was purified as previously described by¹¹.

### Results

### Identification of Pseudomonas putative pore-forming bacteriocin sequences

Putative pore-forming bacteriocins from genus *Pseudomonas* have been retrieved from NCBI by BLAST search using as query pore-forming domain of pyocin S5 (pfam01024). After the analysis of BLAST results we selected six putative pore forming bacteriocins from different *Pseudomonas* species: Pflu095 (*P*. *fluorescens* WP_016979095), Pflu373 (WP_014717373 from strain A506, Pflu794 (WP_081041794 from strain ATCC 17400), Pflu618 (WP_034155618 from strain H16), Pput259 (WP_098964259 from *P. putida* strain FDAARGOS_376) and PmnH from *P. synxantha* strain BG33R.

Clustal W amino acid sequence alignment of pore-forming domains showed 32-49% of identity with pyocin S5 (**Fig. 1A**). The amino acid sequences of pfam 01024 domains of bacteriocins were subjected to phylogenetic tree analysis along with pore-forming domains of some described pore-forming colicins, klebicins and pyocin S5 **(****Fig. 1B****)**. Two major phylogenetic groups could be distinguished: pore forming domains of Pflu794, Pflu373 and Pflu095 are most related to pyocin S5 and belong to the group of colE1-like proteins, and Pflu618 and Put259 are most related to PmnH and belong to colA-like or coIN-like protein group **(****Fig. 1B****).**

### Construction and plant expression of chimeric pyocins

Pore-forming domains of six selected *Pseudomonas* putative porins were used for the construction of the chimeric proteins. All chimeric proteins contain identical N-terminal end of first 309 amino acid residues of pyocin S5 including translocation, FptA binding and CPA binding domains¹⁷. The S5 fragment was fused to the cytotoxic domain of the putative pore-forming bacteriocins. All six chimeric proteins were successfully expressed in *Nicotiana benthamiana* transient expression system and purified by two-step chromatography (**Fig. 2**).

### Example 2: Agar disk-diffusion assay and determination of MIC by agar dilution method

### Agar disk-diffusion assay

Overnight *P. aeruginosa* cultures grown in CAA medium were equalized till OD₅₉₅=1.0 in CAA and diluted 100x. Sterile cotton swab was briefly submerged in diluted microbial suspension, removing the excess of liquid by pressing it against the container wall. The swab was used for evenly streaking bacteria on plates containing growth medium with CAA solid agar (1.5%). 6 mm diameter sterile Whatman discs were placed on soft-agar and 0.3-30 µg of chimeric pyocins were spotted to paper disks. The plates were incubated overnight at 37 °C and bacteriocin inhibition zones were observed.

### Results

A panel of 25 *P. aeruginosa* strains (from culture collections and clinical isolates, **Suppl. Table 1**) were subjected to genomic DNA extraction and PCR analysis using pyocin S5, pyocin S5 immunity protein and FptA receptor-coding gene specific primers. All 25 strains tested positive for the presence of fptA. Six strains (PA14, PAO1, HP6, HP7, ATCC 1960 and NCTC 13921) tested positive for amplification of both pyocin S5 and pyocin S5 immunity protein coding genes (**Fig. 8**).

Next, all 25 *P. aeruginosa* strains were subjected to agar disc-diffusion assay by spotting different amounts of purified pyocin S5 (0.3 µg, 3 µg and 30 µg) on bacteria lawns. Six *P. aeruginosa* strains were resistant to pyocin S5. Four of these six strains were pyocin S5 and immunity protein-encoding strains - PA14, PAO1, ATCC 1960 and NCTC 13921. Two cystic fibrosis isolates, 12-35708 and 12-29165 were also completely resistant to pyocin S5, despite the absence of S5, or S5 immunity protein coding genes. Surprisingly, pyocin S5 still had week inhibition effect on the lawns of two remaining S5 producer strains, clinical isolates HP6 and HP7. Turbid inhibition zones were detected when 3 µg and 30 µg of S5 were spotted on the lawn of these strains (**Table 1**, **Fig 3**).

We analyzed the activity of S5 chimeras in agar disc diffusion assay. Two chimeric proteins, S5-Pflu095 and S5-PmnH demonstrated broadened activity spectrum in comparison to pyocin S5. Both chimeras formed inhibition zones on the lawns of all six S5-producing strains. The inhibition zones on HP6 and HP7 lawns were significantly larger and clearer than those formed by S5. These chimeric proteins also demonstrated activity similar to S5, with only small variations, on all other remaining *P. aeruginosa* strains (**Table 1,** **Fig. 3**). Between the two proteins, S5-PmnH demonstrated slightly superior activity and was selected for further experiments.

Chimeric bacteriocin S5-Pflu373 also demonstrated good activity profile, similar to S5-Pflu095, with exception that it was less active on one of S5 producers, ATCC 19660. S5-Pflu628 and S5-Pput259 chimeras performed less well, and had weaker and less broad activity in comparison to pyocin S5, while S5-Pflu794 demonstrated only week activity on 6 tested strains (**Table 1**, **Fig. 3**).

**Table 1. Activity of chimeric pyocins on panel of P. aeruginosa strains as determined in agar disc-diffusion assay. * - activity detected only with 30 µg of protein, ** - activity detected with 3 µg of protein, *** - activity detected with 0.3 µg of protein. Pyocin S5 killing and immunity protein genes-containing strains are in bold.**

| | **Colicin E1-like** | | | | **Colicin A-like** | | |
|---|---|---|---|---|---|---|---|
| | S5 | S5-Pflu095 | S5-Pflu373 | S5-Pflu794 | S5-PmnH | S5-Pflu618 | S5-Pput259 |
| Boston | *** | *** | *** | * | *** | ** | *** |
| **PA14** | - | ** | ** | - | *** | - | - |
| **PAO1** | - | *** | *** | - | *** | ** | ** |
| Bu002 | *** | *** | *** | - | *** | ** | ** |
| A19 | *** | *** | *** | *** | *** | *** | *** |
| Pr335 | *** | *** | *** | ** | *** | ** | *** |
| EY76 | *** | *** | *** | - | *** | - | - |
| BL77 | *** | ** | ** | - | ** | * | * |
| UR78 | *** | *** | *** | - | *** | * | * |
| BR79 | *** | ** | ** | - | ** | - | * |
| BI80 | *** | *** | *** | - | *** | * | * |
| HP1 | *** | *** | *** | - | *** | ** | *** |
| **HP6** | * | *** | *** | * | *** | * | ** |
| **HP7** | ** | *** | *** | - | *** | ** | ** |
| HP40 | *** | *** | *** | * | *** | ** | ** |
| HP41 | *** | *** | *** | - | *** | ** | ** |
| H P52 | *** | ** | ** | - | ** | * | * |
| HP75 | ** | * | ** | - | ** | - | - |
| ATCC 29260 | *** | *** | *** | - | *** | * | * |
| 12-35708 | - | - | - | - | - | - | - |
| 13-18499 | *** | ** | ** | - | ** | * | * |
| 12-29165 | - | - | - | - | | - | - |
| **ATCC 19660** | - | * | - | - | ** | - | * |
| NCTC 13437 | ** | * | * | - | * | - | - |
| **NCTC 13921** | - | *** | ** | * | *** | * | * |

### Determination of MIC by agar dilution method

Aliquots of melted CAA (1.5% agar) medium (final volume - 25 ml) pre-wormed to 51 °C were supplemented with 0.1 mg/ml BSA and with appropriate amounts of pyocin stock solutions of different concentrations (concentrations lowered by a factor of 2). Aliquots were poured into Petri plates. *P. aeruginosa* cultures were grown from single colony in CAA medium at 37 °C, 200 rpm until OD₅₉₅=0.2 and diluted to 10⁷ CFU/ml in CAA medium. 1 µL of bacteria suspension were applied in three replicates on each test plate. Plates were left to incubate in 37 °C overnight. The determined MIC is a concentration of pyocin, where no confluent bacterial growth is observed.

### Example 3: Ex vivo porcine corneas as model for bacterial keratitis

*Preparation of porcine corneas.* Porcine eyes were acquired from the nearest slaughterhouse. Enucleated eyes were stored at -70 °C. Before the start of experiment, eyes were transferred to 4 °C for 1 hour, then to room temperature for 1-2 hours until the eyeballs were completely defrosted. Then eyeballs were individually placed in sterile plastic containers and submerged for 5 min in 2.5% Povidone-iodine (Betadine 100 mg/ml; EGIS Pharmaceuticals PLC), then twice washed with sterile PBS. The corneas were excised with sterile surgical blade No. #12. The excised corneas were stored in Minimum Essential Medium (MEM) supplemented with Non-Essential Amino Acids, L-Glutamine (2 mM), penicillin (200 U/ml) + spectinomycin (25 µg/ml) until further use at 4 °C up to two weeks.

*Cornea infection and pyocin treatment. P. aeruginosa* was grown overnight from frozen stock. Next morning, the culture was diluted 100 fold by fresh LB medium, and grown until OD595=~0.6 (~6 hours). Bacteria were collected by centrifugation and resuspended in PBS. The dissected corneas were placed on agarose-gelatin (0.5% each) solid support in a 6-well culture plate containing 800 µL MEM with antibiotics (100 U/ml penicillin, 25 µg/ml spectinomycin), then three horizontal and 3 vertical scratches were made using a sterile 25 gauge needle. 3×10⁴ CFU of *P. aeruginosa* ATCC 19660 strain or 0.4×10⁴ CFU of PAO1 strain were applied to cornea and incubated for 16-20 hours in 37 °C CO₂ incubator (20% CO₂). 5 corneas were used for each experimental point. After incubation with *P. aeruginosa,* the corneas were visually inspected for opacity. The corneas which were clear and without signs of infection were considered as non-infected and discarded from further study. 5 µg of S5-PmnH (in 5 µl of PBS) or 5µl of PBS were applied to infected corneas and incubated for additional 16-20 hours in 37 °C CO₂ incubator.

*Homogenization and CFU counting.* Prior the homogenization the corneas were washed 3 times for 10 min in 50 ml sterile PBS with occasional agitation. Each cornea was chopped in 4 equal parts, trying to get rid of sclera. 2 parts were placed in Precellys 24 tissue homogenizer (Bertin technologies) tubes CKMix50-7ml and 2 ml PBS was added. Homogenization was performed at following conditions: 6500 rpm 20 sec., 5 cycles, 3-5 min breaks on ice between the cycles. The obtained homogenate was transferred into 15 ml Falcon tube and briefly spun to sediment the big debris, then the supernatant recovered into new tube and centrifuged at full speed for 10 min to pellet all bacteria. Bacteria were resuspended in 200 µl sterile PBS, serially diluted and plated on LB-agar plates.

### Results

Two types of *P. aeruginosa* strains can be isolated from keratitis cases, cytotoxic *P. aeruginosa* strains mainly cause keratitis related to contact-lens wear, while invasive strains mostly cause disease in post-surgical complications¹⁸. We aimed to investigate if both types of strains could be targeted by S5-PmnH in disease models. Cytotoxic and invasive strains can be distinguished by genotyping the effector protein coding genes of their type III secretion systems (TTSS). Invasive strains were found to possess both exoS and exoT genes, whereas cytotoxic strains appeared to have lost exoS but presented exoT and exoU genes¹⁹⁻²¹. We selected for our experiments cytotoxic strain ATCC 19660 (exoT, exoU) and invasive strain PAO1 (ExoY, exoT, exoS)²². Both strains are pyocin S5 producers and immune to pyocin S5 but both are sensitive to S5-PmnH.

### S5-PmnH treatment can reduce P. aeruginosa bacterial numbers in porcine corneas ex vivo

We first investigated the possibility to use S5-PmnH for eradication of *P. aeruginosa* colonizing the cornea in an ex *vivo* model, the dissected porcine corneas. Porcine corneas were colonized with invasive strain PAO1 or cytotoxic strain ATCC 19660. S5-PmnH MIC determined by agar dilution method against PAO1 was 4 µg/ml and against ATCC 19660 was 32 µg/ml.

In order to obtain *P. aeruginosa* colonization, porcine corneas were incubated with 3×10⁴ CFU of *P. aeruginosa* ATCC 19660 or 0.4×10⁴ CFU of *P. aeruginosa* PAO1 for 16 to 20 hours. Then, 5 µg of S5-PmnH were applied to the cornea and incubated for additional 16-20 hours. At the end of the experiment PAO1 burden in untreated corneas reached an average of 7.6 log₁₀ CFU/cornea, while S5-PmnH-treated corneas the burden was only an average of 10 CFU per cornea, demonstrating 6.6 log₁₀ reduction. In ATCC 19660 colonized corneas, S5-PmnH treatment reduced CFU number by 5.3 log₁₀ (**Fig. 4**). Thus, in ex vivo porcine corneas, S5-PmnH efficiently reduced *P. aeruginosa* colonization by both strains.

### Example 4: Murine keratitis model

### Animals

The inbred mice of C57BL/6 strain of both sexes in equivalent numbers were used for the research. 2-6 months old, adult female and adult male mice were purchased from the Vilnius University vivarium of laboratory animals. The animals throughout the period of the experiment were given standard chow and drinking water *ad libitum.* Animals were housed in the individual plastic cages in a 12 h light/dark cycle at 21-23 °C. All regulated procedures on living animals were approved by The Lithuanian Ethics Committee of Biomedical Research (Protocol no. B1-442) and were carried out in accordance with the European Union legislation of OECD (directive 2010/63/EU). The experiments were carried out in Biological research center of Lithuanian University of Health Sciences.

### P. aeruginosa keratitis induction and treatment

For *P. aeruginosa* infection and keratitis induction the mice were anesthetized by Ketamine and Xylazine 90:9 mg/kg intraperitoneal injection. The cornea of the left eye of each mouse was visualized under a stereoscopic microscope, and three 1 mm scratches were made using a sterile 25 gauge needle. A 10 µL aliquot containing 4×10⁶ cells of *P. aeruginosa* ATCC 19660 (cytotoxic strain) or PAO1 ATCC 15692 (invasive strain) was applied to the corneal surface. Depending on the experiment, the treatment was started 30 min or 6 h post infection. 10 µl aliquot containing 0.14 mg of tobramycin or 20 µg of S5-PmnH (both containing 0.5% hydroxypropyl methylcellulose (HPMC)) or PBS containing 0.5% HPMC was applied as one drop of substance to the each eye. HPMC is used as artificial tears and was used in order to thicken the tears film and prolong the presence of the applied product on the surface of the cornea. The treatment was continued for five days twice daily. Mice were euthanized by cervical dislocation 1, 3, 5 days post infection, and the eyeballs were collected and homogenized for viable bacteria count.

### Assessment of clinical score

The eyes were examined and photographed with a dissection microscope equipped with a digital camera at 1, 3 and 5 dpi to monitor the disease progression. At 1, 3 and 5 dpi disease severity was visually graded by using an established corneal damage scale: 0, the pupil was partially or fully covered by clear or slight opacity; +1, the anterior segment was partially or fully covered by slight opacity; +2, the pupil was partially or fully covered by dense opacity; +3, the entire anterior segment was covered by dense opacity; and +4, corneal perforation⁴⁰.

### Histopathology

Histopathology experiments were carried out at Lithuanian University of Health Sciences, Department of Veterinary Pathobiology of Veterinary Academy. One randomly chosen mice of each study group was used for histopathology experiments. Enucleated eyes were preserved in 10% formaldehyde. Eyes were paraffin-embedded, cut into 3-µm-thick sections, deparaffinised, rehydrated and used for preparation of hematoxylin/eosin stained samples. All samples were observed with the Eclipse TE2000-U microscope (Nikon, Tokyo, Japan).

### Results

### S5-PmnH efficiently kills bacteria and prevents acute disease in murine keratitis models Infection by cytotoxic strain ATCC 19660

For induction of keratitis, the mice were anaesthetized, the cornea of left eye was scratched with a sterile needle and *P. aeruginosa* ATCC 19660 (4×10⁶ CFU) was applied to the corneal surface. The treatment by S5-PmnH, tobramycin or PBS (mock-treatment) was started 30 minutes post infection or 6 h post infection. When the treatment was started 30 min after the infection, no viable *P. aeruginosa* were isolated from infected eyes in both S5-PmnH and tobramycin-treated groups on 1, 3 or 5 dpi. By contrast, the bacterial burden in infected and untreated eyes reached 6-7 log₁₀ CFU/cornea (**Fig. 5A**, left panel).

The visual inspection by microscope of infected untreated eyes revealed acute disease signs: slight to dense opacity of cornea at 1 dpi, and dense opacity and sometimes cornea perforation at 3 dpi. At 5 dpi all the mice had cornea perforations. No signs of disease were observed in majority of samples treated by S5-PmnH or tobramycin (**Fig. 5A**, right panel). The histopathology examination of infected and mock-treated eyes revealed strong corneal inflammation at 1 dpi, and disrupted structure of the eye at 3 dpi and 5 dpi. Only weak oedema in the corneal stroma was observed in all infected eyes treated with PyoS5-PmnH and with tobramycin (**Fig. 5C**.). Thus, both S5-PmnH and tobramycin completely eradicated *P*. *aeruginosa* ATCC 19660 and prevented the disease when the treatment was started almost immediately, 30 min, after infection.

We repeated the experiment with delayed treatment time, allowing the infection to establish for 6 hours. Similar to the previous experiment, the average CFU burden in infected mock-treated eyes reached 6.1 to 6.6 log₁₀ CFU/cornea. In the tobramycin-treated group of mice, no viable bacteria were isolated on1, 3 or 5 dpi. In the S5-PmnH-treated group viable bacteria were isolated from one mouse at 1 dpi (3.6 log₁₀ CFU/cornea) and from one mouse at 3 dpi (5 log₁₀ CFU/cornea). No viable bacteria were isolated from neither of three mice at 5 dpi (**Fig. 5B**, left panel). The clinical score evaluation of infected and mock-treated eyes revealed very similar picture to the previous experiment: the opacity of cornea started at 1 dpi and 3 out of 4 mice had cornea perforations at 5 dpi. Only mild disease signs were observed in two S5-PmnH-treated and one tobramycin-treated mice at 1 and 3 dpi and all corneas were completely clear at 5dpi (**Fig. 5B**, right panel). The histopathology examination of mock-treated eyes revealed the thinning of corneal epithelium, thickening of stroma, acute inflammation at 1 dpi and acute suppurated inflammation of cornea at 3 dpi and 5 dpi. PyoS5-PmnH-treated infected eyes at 1 dpi presented signs of acute inflammation of cornea and no marked aberrations at 3 and 5 dpi, just weak oedema of corneal stroma was observed. Tobramycin-treated infected eyes at 3 and 5 dpi showed slight thickening of epithelium (**Fig. 5C**, right panel). Thus, here again, S5-PmnH treatment was efficient in eradicating *P*. *aeruginosa* from infected corneas and in preventing the establishment and progress of disease.

### Infection by invasive strain PAO1

We examined the efficacy of S5-PmnH for treatment of cornea infection by invasive *P. aeruginosa* PAO1. The infection and treatment procedures were similar to the previous experiment and treatment was started 6 h post infection. The control group of infected mice suffered from severe disease and were euthanized at 3 dpi. At 1 dpi, bacterial burden in mock-treated group of mice reached 6.26 log10 CFU/cornea. The S5-PmnH and tobramycin treatment reduced burden by average 1.04 log10 CFU/cornea and 1.42 log10 CFU/cornea, respectively. At 3 dpi, no viable bacteria were isolated from all three tobramycin-treated mice, and from two S5-PmnH-treated mice. The cornea from the third S5-PmnH-treated mouse contained 2.38 log10 CFU of viable bacteria. By contrast, burden in the control group of mice reached 6.73 log10 CFU/cornea. At 5 dpi, no bacteria were isolated from two tobramycin-treated and one S5-PmnH-treated mice. One mouse from the tobramycin treated group contained 2.8 log10 CFU/cornea. The two remaining mice from the S5-PmnH treated group contained 5.14 log10 and 3.34 log10 CFU/cornea (**Fig. 6A**, left panel).

Clinical examination revealed stronger disease symptoms compared to the cytotoxic strain ATCC 19660. Mock-treated eyes presented severe disease signs at 1 dpi (clinical score 2-3). Several S5-PmnH-treated and tobramycin-treated eyes presented mild clinical scores (1-2) starting from 1 dpi to the end of experiment (**Fig. 6A**, right panel).

Histological examination of uninfected eyes revealed no marked aberrations, although weak edema in corneal stroma was observed in most samples. Infected S5-PmnH-treated eyes presented signs of acute inflammation of cornea at 1 dpi and 3 dpi. Infected tobramycin-treated eyes presented signs of acute inflammation of cornea at 1 dpi, thinning of cornea epithelium and strong edema of the corneal stroma at 3 dpi and local inflammation of cornea, thinning and degeneration of cornea epithelium at day 5 (**Fig. 6C**).

In conclusion, S5-PmnH efficiently reduced bacterial burden and prevented acute disease regardless whether a cytotoxic or invasive strain was used for infection. However, despite of lower S5-PmnH MIC against *P. aeruginosa* PAO1 than against *P. aeruginosa* ATCC 19660, the chimeric pyocin more efficiently eradicated the cytotoxic strain of *P. aeruginosa* and prevented the establishment of disease; a similar effect was observed for tobramycin.

### Example 5: Murine lung colonization and treatment

### Animals

CD1 male mice were supplied by Charles River (Margate, UK) and were specific pathogen free. Mice were 11 to 15 g on receipt at the facility and were allowed to acclimatize for at least 7 days.

Mice were housed in individual ventilated cages exposing the mice at all times to HEPA filtered air. Mice had free access to food and water and were provided with aspen chip bedding.

The room temperature was 22 ± 1°C, with a relative humidity of 60% and maximum background noise of 56 dB. Mice were exposed to 12 hour light/dark cycles.

### Mice infection and pyocin treatment

*P. aeruginosa* ATCC 27853 was recovered from long-term storage (-80°C) and cultured on cystine-lactose-electrolyte-deficient (CLED) agar at 37 °C under aerobic conditions for approximately 16-24 h. 20 ml of Mueller Hinton broth was inoculated with a single well isolated colony and cultured overnight at 37 °C with shaking at 300 rpm65. The overnight broth was diluted 1:100 in Mueller Hinton broth and 100 ml was cultured in a baffle flask for ~6h at 37 °C with shaking at 300 rpm until the broth OD was ~0.6 (~mid-exponential phase). 20 ml of culture was centrifuged at 2465g for 10 minutes and washed in PBS. The pellet was suspended in PBS and the OD₆₀₀ adjusted to 0.67 (~2.6×10⁸ CFU/ml). The study inoculum was prepared from this by appropriate dilution with PBS. The inoculum concentration was confirmed by quantitative culture on *Pseudomonas* Selective Agar (PSA). The inoculum concentration for the study was 1.47 ×10⁷ CFU/ml (5.87 ×10⁵ CFU/mouse).

Mice were infected under temporary inhaled 3% isoflurane anesthesia by intranasal (IN) instillation with 40 µl of the inoculum suspension split as 20 µl/nostril.

1 hour post infection 6 mice were sacrificed in order to evaluate the pre-treatment burden in lungs. Treatments were administered IN once at 1h post infection (50 µl split to 25 µl/nares). Three treatment groups consisting of six mice each were administered S5-PmnH (2.5, 25 and 250 µg). The fourth group of mice (n=6) was administered 200 µg Tobramycin (40 mg/ml injection solution, Hospira UK Ltd, diluted 10 times). The study was terminated at 5 hours post infection for all animals. The clinical conditions and weights were assessed and animals were immediately euthanized using an overdose of pentobarbitone. Following confirmation of death, the lungs were excised and homogenized in ice cold sterile phosphate buffered saline using a Precellys bead beater. The homogenates were quantitatively cultured onto PSA agar and incubated at 37°C for 24 hours before colonies were counted.

### Results

### S5-PmnH efficiently eradicates lung colonization by P. aeruginosa in a murine model of disease

Mice were infected intranasally (IN) with *P. aeruginosa* ATCC 27853 strain. One hour later, 2.5, 25 and 250 µg/mouse of S5-PmnH was administered IN once to both nares of the mouse. 5 hours later mice were euthanized and lung burden of *P. aeruginosa* ATCC 27853 was evaluated.

5 h post infection, *P. aeruginosa* ATCC 27853 burden in the mock-treated mice reached 1.24×10⁷ CFU/g of lung tissue, corresponding to an increase of 1.53 log₁₀ CFU/g from 1 h post infection. S5-PmnH administered IN at 2.5 µg/mouse reduced lung burden by 2.1 log₁₀ CFU/g, administered at 25 µg/mouse by 2.31 log₁₀ CFU/g and administered at 250 µg/mouse by 2.66 log₁₀ CFU/g. The bacterial burden was reduced to below the level of stasis (pre-treatment) in all S5-PmnH treatment groups: in 2.5 µg/mouse group by 0.58 log₁₀ CFU/g, in 25 µg/mouse group by 0.78 log₁₀ CFU/g and in 250 µg/mouse group by 1.13 log₁₀ CFU/g. Increased reduction in burden was observed with higher dose levels of S5-PmnH, however the differences were not statistically significant. Tobramycin administered IN once at 200 µg/mouse reduced bacterial burden by 2.75 log₁₀ CFU/g compared to vehicle, corresponding to 1.23 log₁₀ CFU/g below the level of stasis. Higher variability was observed in this group compared to S5-PmnH or vehicle treatments (Fig. 7).

### General discussion of results

The attempts to use naturally occurring antibacterial proteins for fighting pathogenic bacteria started a while ago. Bacteriophage lysins (endolysins), peptidoglycan hydrolases were extensively exploited in this regard and have been shown to effectively target numerous Gram-positive pathogens ²³, the most advanced of those, lysin PlySs2 (Exebacase), has already entered Phase III clinical trials. Similar to colicin-like bacteriocins, the modular structure of bacteriophage lysins provides an opportunity to engineer enzymes with altered bacteriolytic activity, and several active new hybrid molecules were constructed by swapping the domains of different lysins²⁴. Fighting Gram-negative pathogens, however, is a much greater challenge as outer membrane of the Gram-negative pathogens prevents the access of the potentially antibacterial biologics to the periplasm or bacterial cell cytoplasm and nucleus. Several attempts have been made to enable endolysins to penetrate the outer membrane. For instance, endolysins have been engineered by adding polycathionic or/and hydrophobic/amphipathic peptides to make them able to cross outer membrane (Artilysins)²⁵. As an alternative approach, the attempts have been made to equip colicin-like bacteriocins with lytic domains of endolysins, thus enabling such engineered proteins to translocate to the periplasm of Gram-negative bacteria^{26,27}. In first such attempt, the FyuA binding domain of pesticin, bacteriocin from *Yersinia pestis,* was fused to the N-terminus of T4 lysozyme. This hybrid toxin was shown to kill specific *Yersinia* and pathogenic *E. coli* strains and, importantly, it was able to evade the pesticin immunity protein (Pim) giving it a distinct advantage over pesticin²⁶. In another study, *P. aeruginosa* bacteriocin pyocin S2 domains responsible for surface receptor binding and outer membrane translocation were fused to the GN4 lysin to generate the PyS2-GN4 lysocin. PyS2-GN4 induced peptidoglycan cleavage and log-fold killing of *P. aeruginosa,* efficiently disrupted biofilms, and protected mice from *P. aeruginosa* challenge in a bacteremia model²⁷. However, practical use of this chimeric bacteriocin is limited because of the narrow activity spectrum as it only targets *P. aeruginosa* strains carrying ferripyoverdine receptor FpvAI. These and other previous attempts to engineer new active molecules for control of Gram-negative bacteria were invariably relying on combining domains of phylogenetically and/or functionally unrelated proteins. We therefore decided to construct chimeric molecules by swapping functional domains of closely related bacterial species with the same mechanism of antibacterial activity, namely chimeras of porin-porin type.

The only known pore forming bacteriocin of *P. aeruginosa* was first detected in PAO1 strain²⁸. Pyocin S5 binds the highly conserved ferripyochelin FptA receptor²⁹. The exact prevalence of clinical *P. aeruginosa* strains producing S5 is unknown, but it was demonstrated in the bacteriocin prevalence study of catheter *P. aeruginosa* isolates that about 25% of these strains contained pyocin S5 coding gene³⁰. Thus, these 25% of catheter isolates should be in theory resistant to pyocin S5. We have tested pyocin S5 and S5 immunity protein gene presence in 25 *P. aeruginosa* isolates in our in house collection and found very similar results, 6 strains (or 24%) contained pyocin S5 and immunity protein coding gene sequences. As expected, the presence of pyocin S5 coding gene in these strains correlated with the resistance to the antimicrobial activity of pyocin S5. In our previous study, we also found that pyocin S5 was active against 40% of tested *P. aeruginosa* clinical isolates¹¹. Thus, we speculated that if the immunity of S5 producing strains could be overcome by using cytotoxic domain other than S5, the spectrum of the chimera would be significantly broadened, and it could be able to target more than half of strains.

Towards this goal, we constructed six chimeric S5 pyocins, in which the pore-forming domain of S5 was replaced by pore-forming domains of putative bacteriocins from *Pseudomonas* species other than *P. aeruginosa.* All six chimeric pyocins were successfully expressed in *N. benthamiana* transient expression system, which is our system of choice for expressing colicin-like bacteriocins^{11,31}. Partially purified chimeric proteins were subjected to agar drop test assay on 25 *P. aeruginosa* strains, six between them being producers of S5 and insensitive or only slightly sensitive to this porin.

The chimeric proteins demonstrated variable activity profiles, with three of them standing out as more broadly active than the parental strain S5: two with bacteriocin-like porin fragments coded by *P. fluorescens* (S5-Pflu095 and, S5-Pflu373) and one with pore forming domain from *P. synxantha* bacteriocin (S5-PmnH). PmnH has unusual architecture as it harbors two cytotoxic domains, colicin-M like domain and also pore-forming domain. So far, only activity of its pore-forming domain have been demonstrated¹⁶. The chimeric protein S5-PmnH was active on all six S5-producing strains and did show comparable activity to S5 on all remaining tested *P. aeruginosa* isolates. This chimeric protein was selected for further experiments in murine models for topical treatment of two unrelated models of disease caused by *P. aeruginosa:* keratitis model and lung infection model.

Bacterial keratitis is mostly affecting contact lens wearers, although several other risk factors, such as ocular surface diseases, ocular traumas or ocular surgery, are also not negligible. A 5-year review of cases at Dubai hospital revealed that in 37% of bacterial keratitis cases the causative agent of disease is *P. aeruginosa,* although this frequency may vary in different geographical regions^{32,33}. Untreated bacterial keratitis usually results in blindness.

Two types of *P. aeruginosa* strains are found in eyes affected by keratitis: cytotoxic strains that mainly cause keratitis in contact-lens wearers, and invasive strains mostly causing disease in post-surgical complications¹⁸. Cytotoxic and invasive strains can be distinguished by genotyping the effector protein coding genes of their type III secretion systems (TTSS). Invasive strains were found to possess both exoS and exoT genes whereas cytotoxic strains appeared to have lost exoS but presented exoT and exoU genes¹⁹⁻²¹. In our experiments, we chose cytotoxic strain ATCC 19660 (exoT, exoU) and invasive strain PAO1 (ExoY, exoT, exoS)²². Both strains are pyocin S5 producers and are immune to pyocin S but were confirmed to be sensitive to the chimeric pyocin S5-PmnH.

Chimeric pyocin S5-PmnH demonstrated efficacy in both cytotoxic and invasive *P. aeruginosa* models of eye infection. In the cytotoxic keratitis model, the treatment completely eliminated all bacteria one day post infection, if treatment was started 30 min after infection. When treatment was delayed for 6 hours, viable bacteria were isolated from one mouse at 1 dpi and one mouse at 3 dpi. Visual inspection and histological examination of eyes of all treated mice in both experiments did not reveal any significant lesions, with no marked difference from non-infected eyes. By contrast, very strong keratitis symptoms were observed in all infected eyes at 3 and 5 dpi. Thus, S5-PmnH treatment efficiently eradicated *P. aeruginosa* from cornea infected by the cytotoxic strain *P. aeruginosa* ATCC 19660 and prevented the establishment and progress of the disease.

A similar experiment was performed using the invasive *P. aeruginosa* strain PAO1 with treatment starting 6 hours after infection. Although both treatments (S5-PmnH or tobramycin) were less efficient in eradicating bacteria, the progress of disease was greatly reduced in all treated mice. Taken together, these studies clearly demonstrate the efficacy of chimeric pyocin in this disease model, comparable to that of the standard of care antibiotic tobramycin.

*P. aeruginosa* is also a frequent cause of lung infections, including hospital-acquired pneumonia (HAP) and ventilator-associated pneumonia (VAP). This pathogen has a worsening global trend towards more likely displaying MDR phenotypes³⁴. In addition, *P*. *aeruginosa* can cause chronic lung infections in patients with cystic fibrosis (CF) and non-CF bronchiectasis. Acquisition of *P*. *aeruginosa* is associated with increased morbidity and mortality in patients with CF, and is an important factor in the development and progression of CF respiratory disease³⁵⁻³⁷. Patients with CF are at very high risk of developing infections with multidrug-resistant (MDR) *P. aeruginosa,* owing to the frequent and often prolonged courses of oral, intravenous, and aerosolized antibiotics that are used to treat the chronic lung disease of CF³⁸. In our validated mouse lung colonization model, one single application of chimeric pyocin S5-PmnH, even at the lowest concentration used, 2.5 µg, reduced bacterial burden in the lungs below the level of stasis. The successful results obtained with chimeric pyocin S5-PmnH used in our study show that such bacteriocins can be used for efficiently targeting bacteria in lungs and surprisingly demonstrate engineering bacteriocins with modified or broadened activity spectra.

### AMINO ACID AND NUCLEIC ACIID SEQUENCES

**SEQ ID NO: 1 Pyo S5 first segment** 
MSNDNEVPGSMVIVAQGPDDQYAYEVPPIDSAAVAGNMFGDLIQREIYLQKNIYYPVRSIF
EQGTKEKKEINKKVSDQVDGLLKQITQGKREATRQERVDVMSAVLHKMESDLEGYKKTFT
KGPFIDYEKQSSLSIYEAWVKIWEKNSWEERKKYPFQQLVRDELERAVAYYKQDSLSEAV
KVLRQELNKQKALKEKEDLSQLERDYRTRKANLEMKVQSELDQAGSALPPLVSPTPEQWL
 
ERATRLVTQAIADKKQLQTTNNTLIKNSPTPLEKQKAIYNGELLVDEIASLQARLVKLNAETT
RRRTE
**SEQ ID NO: 2: Pyo S5 first segment coding sequence** 
ATGTCCAATGATAACGAAGTGCCAGGTTCAATGGTTATAGTTGCTCAAGGTCCAGATG
ATCAATACGCTTACGAAGTTCCTCCAATCGATTCTGCTGCTGTTGCTGGAAATATGTTC
GGAGATCTTATCCAAAGGGAAATATATCTTCAAAAGAATATCTATTACCCAGTTAGATCT
ATCTTCGAGCAAGGAACAAAGGAAAAGAAAGAGATTAATAAGAAAGTTTCAGATCAAGT
TGATGGACTTTTGAAGCAAATTACACAAGGAAAGAGAGAAGCTACTAGGCAAGAGAGA
GTTGATGTTATGTCAGCTGTTCTTCATAAGATGGAATCTGATTTGGAGGGATATAAGAA
AACTTTTACAAAGGGTCCTTTTATTGATTACGAAAAGCAATCTTCACTTTCAATCTATGA
AGCTTGGGTTAAGATTTGGGAGAAAAATTCTTGGGAGGAGAGAAAGAAATACCCTTTT
CAACAACTTGTTAGGGATGAATTGGAGAGAGCTGTTGCTTATTACAAGCAAGATTCTCT
TTCAGAAGCTGTTAAAGTTCTTAGGCAAGAGTTGAATAAGCAAAAGGCTTTGAAGGAAA
AGGAGGATCTTTCACAATTGGAAAGAGATTACAGGACAAGAAAGGCTAATCTTGAGAT
GAAGGTTCAATCTGAGTTGGATCAAGCTGGATCAGCTCTTCCACCTTTGGTTTCTCCAA
CTCCTGAACAATGGCTTGAGAGGGCTACAAGATTGGTTACTCAAGCTATTGCTGATAA
GAAACAACTTCAAACTACAAATAACACATTGATTAAGAACTCTCCAACTCCTCTTGAAAA
GCAAAAAGCTATCTATAATGGTGAACTTTTGGTTGATGAGATTGCTTCATTGCAAGCTA
GGCTTGTTAAATTGAATGCTGAGACTACAAGAAGGAGAACAGAA
**SEQ ID NO: 3: PmnH second segment** 
KELTPDEKAAYTDAVSFVSTANEQMLQKYGANLSKVAQDMQAEIAGKKVRSYAEAMATFE
KISANPAMKLNALDTQAVVDALNALDKASFADNITRLGKAFGVVGKVVQAEAIREKTVSGF
QTGDWKPLMLELEAMAVGTGAGILLATSMAFFFPVFASAAAGVVVVALMMAATAAYFDAA
KVDEINNLILN
**SEQ ID NO: 4: PmnH second segment coding sequence** 
AAGGAACTTACACCAGATGAGAAAGCTGCTTATACTGATGCTGTTTCTTTTGTTTCAAC
AGCTAATGAGCAAATGTTGCAAAAGTACGGTGCTAATCTTTCAAAAGTTGCTCAAGATA
TGCAAGCTGAGATTGCTGGAAAGAAAGTTAGGTCTTATGCTGAAGCTATGGCTACTTTT
GAGAAGATTTCAGCTAATCCAGCTATGAAACTTAATGCTTTGGATACACAAGCTGTTGT
TGATGCTCTTAATGCTTTGGATAAGGCTTCTTTCGCTGATAACATCACTAGGTTGGGAA
AGGCTTTTGGTGTTGTTGGAAAAGTTGTTCAAGCTGAAGCTATTAGAGAGAAGACAGTT
TCAGGTTTTCAAACTGGAGATTGGAAACCTTTGATGCTTGAATTGGAGGCTATGGCTGT
TGGAACTGGTGCTGGAATTCTTTTGGCTACATCTATGGCTTTCTTTTTCCCAGTTTTTG
CTTCAGCTGCTGCTGGAGTTGTTGTTGTTGCTCTTATGATGGCTGCTACAGCTGCTTAC
TTTGATGCTGCTAAGGTTGATGAAATTAATAACCTTATTTTGAATTAA
**SEQ ID NO: 5: S5-PmnH,** pyocin S5 part is underlined. 
MSNDNEVPGSMVIVAQGPDDQYAYEVPPIDSAAVAGNMFGDLIQREIYLQKNIYYPVRSIF
EQGTKEKKEINKKVSDQVDGLLKQITQGKREATRQERVDVMSAVLHKMESDLEGYKKTFT
KGPFIDYEKQSSLSIYEAWVKIWEKNSWEERKKYPFQQLVRDELERAVAYYKQDSLSEAV
KVLRQELNKQKALKEKEDLSQLERDYRTRKANLEMKVQSELDQAGSALPPLVSPTPEQWL
ERATRLVTQAIADKKQLQTTNNTLIKNSPTPLEKQKAIYNGELLVDEIASLQARLVKLNAETT
RRRTEKELTPDEKAAYTDAVSFVSTANEQMLQKYGANLSKVAQDMQAEIAGKKVRSYAEA
MATFEKISANPAMKLNALDTQAVVDALNALDKASFADNITRLGKAFGVVGKVVQAEAIREK
 
TVSGFQTGDWKPLMLELEAMAVGTGAGILLATSMAFFFPVFASAAAGVVVVALMMAATAA
YFDAAKVDEINNLILN
**SEQ ID NO: 6: S5-PmnH coding sequence,** pyocin S5 part is underlined 
ATGTCCAATGATAACGAAGTGCCAGGTTCAATGGTTATAGTTGCTCAAGGTCCAGATG
ATCAATACGCTTACGAAGTTCCTCCAATCGATTCTGCTGCTGTTGCTGGAAATATGTTC
GGAGATCTTATCCAAAGGGAAATATATCTTCAAAAGAATATCTATTACCCAGTTAGATCT
ATCTTCGAGCAAGGAACAAAGGAAAAGAAAGAGATTAATAAGAAAGTTTCAGATCAAGT
TGATGGACTTTTGAAGCAAATTACACAAGGAAAGAGAGAAGCTACTAGGCAAGAGAGA
GTTGATGTTATGTCAGCTGTTCTTCATAAGATGGAATCTGATTTGGAGGGATATAAGAA
AACTTTTACAAAGGGTCCTTTTATTGATTACGAAAAGCAATCTTCACTTTCAATCTATGA
AGCTTGGGTTAAGATTTGGGAGAAAAATTCTTGGGAGGAGAGAAAGAAATACCCTTTT
CAACAACTTGTTAGGGATGAATTGGAGAGAGCTGTTGCTTATTACAAGCAAGATTCTCT
TTCAGAAGCTGTTAAAGTTCTTAGGCAAGAGTTGAATAAGCAAAAGGCTTTGAAGGAAA
AGGAGGATCTTTCACAATTGGAAAGAGATTACAGGACAAGAAAGGCTAATCTTGAGAT
GAAGGTTCAATCTGAGTTGGATCAAGCTGGATCAGCTCTTCCACCTTTGGTTTCTCCAA
CTCCTGAACAATGGCTTGAGAGGGCTACAAGATTGGTTACTCAAGCTATTGCTGATAA
GAAACAACTTCAAACTACAAATAACACATTGATTAAGAACTCTCCAACTCCTCTTGAAAA
GCAAAAAGCTATCTATAATGGTGAACTTTTGGTTGATGAGATTGCTTCATTGCAAGCTA
GGCTTGTTAAATTGAATGCTGAGACTACAAGAAGGAGAACAGAAAAGGAACTTACACC
AGATGAGAAAGCTGCTTATACTGATGCTGTTTCTTTTGTTTCAACAGCTAATGAGCAAA
TGTTGCAAAAGTACGGTGCTAATCTTTCAAAAGTTGCTCAAGATATGCAAGCTGAGATT
GCTGGAAAGAAAGTTAGGTCTTATGCTGAAGCTATGGCTACTTTTGAGAAGATTTCAGC
TAATCCAGCTATGAAACTTAATGCTTTGGATACACAAGCTGTTGTTGATGCTCTTAATG
CTTTGGATAAGGCTTCTTTCGCTGATAACATCACTAGGTTGGGAAAGGCTTTTGGTGTT
GTTGGAAAAGTTGTTCAAGCTGAAGCTATTAGAGAGAAGACAGTTTCAGGTTTTCAAAC
TGGAGATTGGAAACCTTTGATGCTTGAATTGGAGGCTATGGCTGTTGGAACTGGTGCT
GGAATTCTTTTGGCTACATCTATGGCTTTCTTTTTCCCAGTTTTTGCTTCAGCTGCTGCT
GGAGTTGTTGTTGTTGCTCTTATGATGGCTGCTACAGCTGCTTACTTTGATGCTGCTAA
GGTTGATGAAATTAATAACCTTATTTTGAATTAA
**SEQ ID NO: 7: Pflu095 second segment** 
KDKVRTEVVDIEAAIKFTSDFYAEAGVRFGDSASKLASDLADSAQGKKIRSADEAFKAFDK
YKNELDKKFSVKDRAAAAKYIDSIDYEAIGKAATKFSKGLGYVGPVIDAKDSIIEFINSMESG
DWKPFFLKLESIALGLAATAFVGIAFGFIATTPMGILAFAFIVAATGAAIDDNFAEKLNKFVSS
L
**SEQ ID NO: 8: Pflu095 second segment coding sequence** 
AAGGATAAGGTTAGGACTGAGGTGGTGGACATCGAGGCTGCTATTAAGTTCACCAGC
GACTTCTATGCTGAGGCTGGTGTTAGGTTCGGCGACTCTGCTTCTAAGCTTGCTAGCG
ATCTTGCTGATAGCGCCCAGGGTAAGAAAATCAGGTCTGCTGATGAGGCTTTCAAGGC
CTTCGACAAGTACAAGAACGAGCTGGACAAGAAGTTCAGCGTCAAGGATAGAGCTGCT
GCCGCTAAGTACATCGACAGCATTGATTACGAGGCTATCGGCAAGGCTGCCACCAAG
TTCTCTAAAGGTCTTGGTTACGTGGGCCCTGTGATCGATGCTAAGGATAGCATCATCG
AGTTCATCAACAGCATGGAAAGCGGCGACTGGAAGCCATTCTTCCTGAAGCTTGAGTC
TATCGCTCTGGGTCTTGCTGCTACTGCTTTCGTGGGTATTGCCTTCGGTTTCATTGCTA
 
CTACCCCTATGGGTATCCTGGCCTTCGCTTTTATTGTGGCTGCTACCGGTGCTGCTAT
CGACGATAATTTCGCTGAGAAGCTGAACAAGTTCGTGTCCAGCCTTTAA
**SEQ ID NO: 9: S5-Pflu095**, pyocin S5 part is underlined 
MSNDNEVPGSMVIVAQGPDDQYAYEVPPIDSAAVAGNMFGDLIQREIYLQKNIYYPVRSIF
EQGTKEKKEINKKVSDQVDGLLKQITQGKREATRQERVDVMSAVLHKMESDLEGYKKTFT
KGPFIDYEKQSSLSIYEAWVKIWEKNSWEERKKYPFQQLVRDELERAVAYYKQDSLSEAV
KVLRQELNKQKALKEKEDLSQLERDYRTRKANLEMKVQSELDQAGSALPPLVSPTPEQWL
ERATRLVTQAIADKKQLQTTNNTLIKNSPTPLEKQKAIYNGELLVDEIASLQARLVKLNAETT
RRRTEKDKVRTEVVDIEAAIKFTSDFYAEAGVRFGDSASKLASDLADSAQGKKIRSADEAF
KAFDKYKNELDKKFSVKDRAAAAKYIDSIDYEAIGKAATKFSKGLGYVGPVIDAKDSIIEFINS
MESGDWKPFFLKLESIALGLAATAFVGIAFGFIATTPMGILAFAFIVAATGAAIDDNFAEKLNK
FVSSL
**SEQ ID NO: 10: S5-Pflu095 coding sequence**, pyocin S5 part is underlined 
ATGTCCAATGATAACGAAGTGCCAGGTTCAATGGTTATAGTTGCTCAAGGTCCAGATG
ATCAATACGCTTACGAAGTTCCTCCAATCGATTCTGCTGCTGTTGCTGGAAATATGTTC
GGAGATCTTATCCAAAGGGAAATATATCTTCAAAAGAATATCTATTACCCAGTTAGATCT
ATCTTCGAGCAAGGAACAAAGGAAAAGAAAGAGATTAATAAGAAAGTTTCAGATCAAGT
TGATGGACTTTTGAAGCAAATTACACAAGGAAAGAGAGAAGCTACTAGGCAAGAGAGA
GTTGATGTTATGTCAGCTGTTCTTCATAAGATGGAATCTGATTTGGAGGGATATAAGAA
AACTTTTACAAAGGGTCCTTTTATTGATTACGAAAAGCAATCTTCACTTTCAATCTATGA
AGCTTGGGTTAAGATTTGGGAGAAAAATTCTTGGGAGGAGAGAAAGAAATACCCTTTT
CAACAACTTGTTAGGGATGAATTGGAGAGAGCTGTTGCTTATTACAAGCAAGATTCTCT
TTCAGAAGCTGTTAAAGTTCTTAGGCAAGAGTTGAATAAGCAAAAGGCTTTGAAGGAAA
AGGAGGATCTTTCACAATTGGAAAGAGATTACAGGACAAGAAAGGCTAATCTTGAGAT
GAAGGTTCAATCTGAGTTGGATCAAGCTGGATCAGCTCTTCCACCTTTGGTTTCTCCAA
CTCCTGAACAATGGCTTGAGAGGGCTACAAGATTGGTTACTCAAGCTATTGCTGATAA
GAAACAACTTCAAACTACAAATAACACATTGATTAAGAACTCTCCAACTCCTCTTGAAAA
GCAAAAAGCTATCTATAATGGTGAACTTTTGGTTGATGAGATTGCTTCATTGCAAGCTA
GGCTTGTTAAATTGAATGCTGAGACTACAAGAAGGAGAACAGAAAAGGATAAGGTTAG
GACTGAGGTGGTGGACATCGAGGCTGCTATTAAGTTCACCAGCGACTTCTATGCTGAG
GCTGGTGTTAGGTTCGGCGACTCTGCTTCTAAGCTTGCTAGCGATCTTGCTGATAGCG
CCCAGGGTAAGAAAATCAGGTCTGCTGATGAGGCTTTCAAGGCCTTCGACAAGTACAA
GAACGAGCTGGACAAGAAGTTCAGCGTCAAGGATAGAGCTGCTGCCGCTAAGTACAT
CGACAGCATTGATTACGAGGCTATCGGCAAGGCTGCCACCAAGTTCTCTAAAGGTCTT
GGTTACGTGGGCCCTGTGATCGATGCTAAGGATAGCATCATCGAGTTCATCAACAGCA
TGGAAAGCGGCGACTGGAAGCCATTCTTCCTGAAGCTTGAGTCTATCGCTCTGGGTCT
TGCTGCTACTGCTTTCGTGGGTATTGCCTTCGGTTTCATTGCTACTACCCCTATGGGTA
TCCTGGCCTTCGCTTTTATTGTGGCTGCTACCGGTGCTGCTATCGACGATAATTTCGCT
GAGAAGCTGAACAAGTTCGTGTCCAGCCTTTAA
**SEQ ID NO: 11: Pflu373 second segment** 
AEEQAKAEEEIKGAIKFTADFYKEIGEKYGAQMTASATDLAETAKGKTLRSAEEALKAFDQY
KDHLDKKFSAADRAAIVNALDSLDRAELAKNLNLFAKGFGYTSKAFDVYDLVEEVKKSYAS
GDWNNTALKVETLFAGSAATGLIAFAFGVTVSTPVGIVAFALIMALVSAYIDDAHVKQFNDA
LDAILP
**SEQ ID NO: 12: Pflu373 second segment coding sequence** 
GCTGAGGAACAGGCTAAGGCTGAGGAAGAGATTAAGGGCGCTATCAAGTTCACCGCC
GACTTCTACAAAGAAATCGGCGAGAAGTACGGCGCTCAGATGACTGCTTCTGCTACTG
ATCTTGCTGAGACTGCTAAGGGTAAGACCCTTAGGTCAGCAGAAGAGGCTCTGAAGG
CTTTCGATCAGTACAAGGATCACCTGGACAAGAAGTTCAGCGCTGCTGATAGGGCTGC
TATTGTGAACGCTCTGGATTCTCTTGACAGGGCTGAGCTTGCTAAGAACCTGAACCTG
TTCGCTAAAGGCTTCGGCTACACCTCTAAGGCATTCGATGTGTACGATCTGGTGGAAG
AGGTGAAGAAAAGCTACGCTAGCGGCGATTGGAACAACACCGCTCTTAAGGTTGAGA
CTCTGTTCGCTGGTTCTGCTGCTACCGGTTTGATTGCTTTCGCTTTCGGTGTGACTGT
GTCTACCCCTGTTGGTATTGTGGCTTTCGCTCTGATTATGGCTCTGGTGAGCGCTTAC
ATCGATGACGCTCATGTGAAGCAGTTCAACGATGCTCTGGATGCTATCCTGCCTTAA
**SEQ ID NO: 13: S5-Pflu373,** pyocin S5 part is underlined 
MSNDNEVPGSMVIVAQGPDDQYAYEVPPIDSAAVAGNMFGDLIQREIYLQKNIYYPVRSIF
EQGTKEKKEINKKVSDQVDGLLKQITQGKREATRQERVDVMSAVLHKMESDLEGYKKTFT
KGPFIDYEKQSSLSIYEAWVKIWEKNSWEERKKYPFQQLVRDELERAVAYYKQDSLSEAV
KVLRQELNKQKALKEKEDLSQLERDYRTRKANLEMKVQSELDQAGSALPPLVSPTPEQWL
ERATRLVTQAIADKKQLQTTNNTLIKNSPTPLEKQKAIYNGELLVDEIASLQARLVKLNAETT
RRRTEAEEQAKAEEEIKGAIKFTADFYKEIGEKYGAQMTASATDLAETAKGKTLRSAEEALK
AFDQYKDHLDKKFSAADRAAIVNALDSLDRAELAKNLNLFAKGFGYTSKAFDVYDLVEEVK
KSYASGDWNNTALKVETLFAGSAATGLIAFAFGVTVSTPVGIVAFALIMALVSAYIDDAHVK
QFNDALDAILP
**SEQ ID NO: 14: S5-Pflu373 coding sequence,** pyocin S5 part is underlined 
ATGTCCAATGATAACGAAGTGCCAGGTTCAATGGTTATAGTTGCTCAAGGTCCAGATG
ATCAATACGCTTACGAAGTTCCTCCAATCGATTCTGCTGCTGTTGCTGGAAATATGTTC
GGAGATCTTATCCAAAGGGAAATATATCTTCAAAAGAATATCTATTACCCAGTTAGATCT
ATCTTCGAGCAAGGAACAAAGGAAAAGAAAGAGATTAATAAGAAAGTTTCAGATCAAGT
TGATGGACTTTTGAAGCAAATTACACAAGGAAAGAGAGAAGCTACTAGGCAAGAGAGA
GTTGATGTTATGTCAGCTGTTCTTCATAAGATGGAATCTGATTTGGAGGGATATAAGAA
AACTTTTACAAAGGGTCCTTTTATTGATTACGAAAAGCAATCTTCACTTTCAATCTATGA
AGCTTGGGTTAAGATTTGGGAGAAAAATTCTTGGGAGGAGAGAAAGAAATACCCTTTT
CAACAACTTGTTAGGGATGAATTGGAGAGAGCTGTTGCTTATTACAAGCAAGATTCTCT
TTCAGAAGCTGTTAAAGTTCTTAGGCAAGAGTTGAATAAGCAAAAGGCTTTGAAGGAAA
AGGAGGATCTTTCACAATTGGAAAGAGATTACAGGACAAGAAAGGCTAATCTTGAGAT
GAAGGTTCAATCTGAGTTGGATCAAGCTGGATCAGCTCTTCCACCTTTGGTTTCTCCAA
CTCCTGAACAATGGCTTGAGAGGGCTACAAGATTGGTTACTCAAGCTATTGCTGATAA
GAAACAACTTCAAACTACAAATAACACATTGATTAAGAACTCTCCAACTCCTCTTGAAAA
GCAAAAAGCTATCTATAATGGTGAACTTTTGGTTGATGAGATTGCTTCATTGCAAGCTA
GGCTTGTTAAATTGAATGCTGAGACTACAAGAAGGAGAACAGAAGCTGAGGAACAGGC
TAAGGCTGAGGAAGAGATTAAGGGCGCTATCAAGTTCACCGCCGACTTCTACAAAGAA
ATCGGCGAGAAGTACGGCGCTCAGATGACTGCTTCTGCTACTGATCTTGCTGAGACTG
CTAAGGGTAAGACCCTTAGGTCAGCAGAAGAGGCTCTGAAGGCTTTCGATCAGTACAA
GGATCACCTGGACAAGAAGTTCAGCGCTGCTGATAGGGCTGCTATTGTGAACGCTCT
GGATTCTCTTGACAGGGCTGAGCTTGCTAAGAACCTGAACCTGTTCGCTAAAGGCTTC
GGCTACACCTCTAAGGCATTCGATGTGTACGATCTGGTGGAAGAGGTGAAGAAAAGCT
 
ACGCTAGCGGCGATTGGAACAACACCGCTCTTAAGGTTGAGACTCTGTTCGCTGGTTC
TGCTGCTACCGGTTTGATTGCTTTCGCTTTCGGTGTGACTGTGTCTACCCCTGTTGGTA
TTGTGGCTTTCGCTCTGATTATGGCTCTGGTGAGCGCTTACATCGATGACGCTCATGT
GAAGCAGTTCAACGATGCTCTGGATGCTATCCTGCCTTAA
**SEQ ID NO: 15: Pflu794 second segment** 
KASDQANNVDIEEAILFTTTFYENLTEKYGEKVSAVAKELAESAKGKTMRSSKEALQTFEKY
KDTYNGRFRSRDRREVDRALKSLDKELLSKNLAKFSKAFGSVSKIGDLTEVFIELENSIRTG
DWKPLLLTLEGIGLGMAGTYLVAAVFGISATTPLGIVVFAVLMAATSAYIDDDLVKKVNKDLF
GF
**SEQ ID NO: 16: Pflu794 second segment coding sequence** 
AAGGCTAGCGACCAGGCTAACAACGTTGACATCGAAGAGGCTATCCTGTTCACCACCA
CCTTCTACGAGAACCTCACTGAGAAGTACGGCGAGAAGGTTAGCGCTGTGGCTAAAG
AACTTGCTGAGAGCGCTAAGGGCAAGACCATGAGGTCATCTAAAGAGGCTCTGCAGA
CCTTCGAGAAGTACAAGGATACCTACAACGGCCGGTTCAGGTCTAGGGATAGAAGGG
AAGTTGATCGGGCTCTGAAGTCCCTTGACAAAGAGCTGCTGTCTAAGAACCTCGCCAA
GTTCTCTAAGGCTTTCGGCTCCGTGTCTAAGATCGGTGATCTTACCGAGGTGTTCATC
GAGTTGGAGAACTCTATCAGGACCGGCGATTGGAAGCCTCTTCTGCTTACCCTTGAAG
GTATCGGTCTTGGTATGGCTGGAACCTACCTTGTTGCTGCTGTGTTCGGTATCTCTGC
TACTACCCCTCTTGGCATTGTGGTGTTCGCTGTTCTTATGGCTGCTACCAGCGCTTACA
TCGATGATGACCTGGTGAAGAAGGTGAACAAGGACCTGTTCGGCTTCTAA
**SEQ ID NO: 17: S5-Pflu794,** pyocin S5 part is underlined 
MSNDNEVPGSMVIVAQGPDDQYAYEVPPIDSAAVAGNMFGDLIQREIYLQKNIYYPVRSIF
EQGTKEKKEINKKVSDQVDGLLKQITQGKREATRQERVDVMSAVLHKMESDLEGYKKTFT
KGPFIDYEKQSSLSIYEAWVKIWEKNSWEERKKYPFQQLVRDELERAVAYYKQDSLSEAV
KVLRQELNKQKALKEKEDLSQLERDYRTRKANLEMKVQSELDQAGSALPPLVSPTPEQWL
ERATRLVTQAIADKKQLQTTNNTLIKNSPTPLEKQKAIYNGELLVDEIASLQARLVKLNAETT
RRRTEKASDQANNVDIEEAILFTTTFYENLTEKYGEKVSAVAKELAESAKGKTMRSSKEAL
QTFEKYKDTYNGRFRSRDRREVDRALKSLDKELLSKNLAKFSKAFGSVSKIGDLTEVFIELE
NSIRTGDWKPLLLTLEGIGLGMAGTYLVAAVFGISATTPLGIVVFAVLMAATSAYIDDDLVKK
VNKDLFGF
**SEQ ID NO: 18: S5-Pflu794 coding sequence,** pyocin S5 part is underlined 
ATGTCCAATGATAACGAAGTGCCAGGTTCAATGGTTATAGTTGCTCAAGGTCCAGATG
ATCAATACGCTTACGAAGTTCCTCCAATCGATTCTGCTGCTGTTGCTGGAAATATGTTC
GGAGATCTTATCCAAAGGGAAATATATCTTCAAAAGAATATCTATTACCCAGTTAGATCT
ATCTTCGAGCAAGGAACAAAGGAAAAGAAAGAGATTAATAAGAAAGTTTCAGATCAAGT
TGATGGACTTTTGAAGCAAATTACACAAGGAAAGAGAGAAGCTACTAGGCAAGAGAGA
GTTGATGTTATGTCAGCTGTTCTTCATAAGATGGAATCTGATTTGGAGGGATATAAGAA
AACTTTTACAAAGGGTCCTTTTATTGATTACGAAAAGCAATCTTCACTTTCAATCTATGA
AGCTTGGGTTAAGATTTGGGAGAAAAATTCTTGGGAGGAGAGAAAGAAATACCCTTTT
CAACAACTTGTTAGGGATGAATTGGAGAGAGCTGTTGCTTATTACAAGCAAGATTCTCT
TTCAGAAGCTGTTAAAGTTCTTAGGCAAGAGTTGAATAAGCAAAAGGCTTTGAAGGAAA
AGGAGGATCTTTCACAATTGGAAAGAGATTACAGGACAAGAAAGGCTAATCTTGAGAT
GAAGGTTCAATCTGAGTTGGATCAAGCTGGATCAGCTCTTCCACCTTTGGTTTCTCCAA
 
CTCCTGAACAATGGCTTGAGAGGGCTACAAGATTGGTTACTCAAGCTATTGCTGATAA
GAAACAACTTCAAACTACAAATAACACATTGATTAAGAACTCTCCAACTCCTCTTGAAAA
GCAAAAAGCTATCTATAATGGTGAACTTTTGGTTGATGAGATTGCTTCATTGCAAGCTA
GGCTTGTTAAATTGAATGCTGAGACTACAAGAAGGAGAACAGAAAAGGCTAGCGACCA
GGCTAACAACGTTGACATCGAAGAGGCTATCCTGTTCACCACCTTCTACGAGAAC
CTCACTGAGAAGTACGGCGAGAAGGTTAGCGCTGTGGCTAAAGAACTTGCTGAGAGC
GCTAAGGGCAAGACCATGAGGTCATCTAAAGAGGCTCTGCAGACCTTCGAGAAGTAC
AAGGATACCTACAACGGCCGGTTCAGGTCTAGGGATAGAAGGGAAGTTGATCGGGCT
CTGAAGTCCCTTGACAAAGAGCTGCTGTCTAAGAACCTCGCCAAGTTCTCTAAGGCTT
TCGGCTCCGTGTCTAAGATCGGTGATCTTACCGAGGTGTTCATCGAGTTGGAGAACTC
TATCAGGACCGGCGATTGGAAGCCTCTTCTGCTTACCCTTGAAGGTATCGGTCTTGGT
ATGGCTGGAACCTACCTTGTTGCTGCTGTGTTCGGTATCTCTGCTACTACCCCTCTTG
GCATTGTGGTGTTCGCTGTTCTTATGGCTGCTACCAGCGCTTACATCGATGATGACCT
GGTGAAGAAGGTGAACAAGGACCTGTTCGGCTTCTAA
**SEQ ID NO: 19: Pflu618 second segment** 
AEAEAKRIDDYKRAVAFVADANKYILEKYGANLHQVVMDLQKDISGKKIRSYAEAMQTFEA
VRTNPNARLSPQDTRAVVDALNALDKATYMDSVNKLAKGFGVTGKIVQAHSVIEKTVIGFR
DGNWKPLILELESIALGAGAGAAVATLLAVFSPGFAASAIGIVAVGVAIATIASLLNADNVEKI
NVFISDHLETALKEQR
**SEQ ID NO: 20: Pflu618 second segment coding sequence** 
GCTGAAGCTGAGGCTAAGAGAATTGATGATTACAAAAGGGCTGTTGCTTTCGTTGCTG
ATGCTAATAAGTACATCTTGGAAAAGTACGGTGCTAATCTTCATCAAGTTGTTATGGAT
TTGCAAAAGGATATCTCTGGAAAGAAAATTAGATCATACGCTGAAGCTATGCAAACTTT
TGAGGCTGTTAGAACAAATCCAAATGCTAGGCTTTCTCCTCAAGATACTAGGGCTGTT
GTTGATGCTTTGAATGCTCTTGATAAGGCTACATACATGGATTCTGTTAATAAGTTGGC
TAAAGGATTTGGTGTTACTGGAAAGATTGTTCAAGCTCATTCAGTTATTGAAAAGACAG
TTATTGGTTTTAGAGATGGAAATTGGAAACCACTTATTTTGGAACTTGAGTCAATTGCTT
TGGGAGCTGGTGCTGGAGCTGCTGTTGCTACACTTTTGGCTGTTTTCTCTCCTGGATT
TGCTGCTTCAGCTATTGGTATTGTTGCTGTTGGAGTTGCTATTGCTACTATTGCTTCTC
TTTTGAACGCTGATAACGTTGAGAAGATTAATGTTTTTATTTCAGATCATTTGGAAACAG
CTCTTAAAGAGCAAAGGTAA
**SEQ ID NO: 21: S5-Pflu618,** pyocin S5 part is underlined 
MSNDNEVPGSMVIVAQGPDDQYAYEVPPIDSAAVAGNMFGDLIQREIYLQKNIYYPVRSIF
EQGTKEKKEINKKVSDQVDGLLKQITQGKREATRQERVDVMSAVLHKMESDLEGYKKTFT
KGPFIDYEKQSSLSIYEAWVKIWEKNSWEERKKYPFQQLVRDELERAVAYYKQDSLSEAV
KVLRQELNKQKALKEKEDLSQLERDYRTRKANLEMKVQSELDQAGSALPPLVSPTPEQWL
ERATRLVTQAIADKKQLQTTNNTLIKNSPTPLEKQKAIYNGELLVDEIASLQARLVKLNAETT
RRRTEAEAEAKRIDDYKRAVAFVADANKYILEKYGANLHQVVMDLQKDISGKKIRSYAEAM
QTFEAVRTNPNARLSPQDTRAVVDALNALDKATYMDSVNKLAKGFGVTGKIVQAHSVIEKT
VIGFRDGNWKPLILELESIALGAGAGAAVATLLAVFSPGFAASAIGIVAVGVAIATIASLLNAD
NVEKINVFISDHLETALKEQR
**SEQ ID NO: 22: S5-Pflu618 coding sequence**, pyocin S5 part is underlined 
ATGTCCAATGATAACGAAGTGCCAGGTTCAATGGTTATAGTTGCTCAAGGTCCAGATG
ATCAATACGCTTACGAAGTTCCTCCAATCGATTCTGCTGCTGTTGCTGGAAATATGTTC
GGAGATCTTATCCAAAGGGAAATATATCTTCAAAAGAATATCTATTACCCAGTTAGATCT
ATCTTCGAGCAAGGAACAAAGGAAAAGAAAGAGATTAATAAGAAAGTTTCAGATCAAGT
TGATGGACTTTTGAAGCAAATTACACAAGGAAAGAGAGAAGCTACTAGGCAAGAGAGA
GTTGATGTTATGTCAGCTGTTCTTCATAAGATGGAATCTGATTTGGAGGGATATAAGAA
AACTTTTACAAAGGGTCCTTTTATTGATTACGAAAAGCAATCTTCACTTTCAATCTATGA
AGCTTGGGTTAAGATTTGGGAGAAAAATTCTTGGGAGGAGAGAAAGAAATACCCTTTT
CAACAACTTGTTAGGGATGAATTGGAGAGAGCTGTTGCTTATTACAAGCAAGATTCTCT
TTCAGAAGCTGTTAAAGTTCTTAGGCAAGAGTTGAATAAGCAAAAGGCTTTGAAGGAAA
AGGAGGATCTTTCACAATTGGAAAGAGATTACAGGACAAGAAAGGCTAATCTTGAGAT
GAAGGTTCAATCTGAGTTGGATCAAGCTGGATCAGCTCTTCCACCTTTGGTTTCTCCAA
CTCCTGAACAATGGCTTGAGAGGGCTACAAGATTGGTTACTCAAGCTATTGCTGATAA
GAAACAACTTCAAACTACAAATAACACATTGATTAAGAACTCTCCAACTCCTCTTGAAAA
GCAAAAAGCTATCTATAATGGTGAACTTTTGGTTGATGAGATTGCTTCATTGCAAGCTA
GGCTTGTTAAATTGAATGCTGAGACTACAAGAAGGAGAACAGAAGCTGAAGCTGAGGC
TAAGAGAATTGATGATTACAAAAGGGCTGTTGCTTTCGTTGCTGATGCTAATAAGTACA
TCTTGGAAAAGTACGGTGCTAATCTTCATCAAGTTGTTATGGATTTGCAAAAGGATATC
TCTGGAAAGAAAATTAGATCATACGCTGAAGCTATGCAAACTTTTGAGGCTGTTAGAAC
AAATCCAAATGCTAGGCTTTCTCCTCAAGATACTAGGGCTGTTGTTGATGCTTTGAATG
CTCTTGATAAGGCTACATACATGGATTCTGTTAATAAGTTGGCTAAAGGATTTGGTGTT
ACTGGAAAGATTGTTCAAGCTCATTCAGTTATTGAAAAGACAGTTATTGGTTTTAGAGA
TGGAAATTGGAAACCACTTATTTTGGAACTTGAGTCAATTGCTTTGGGAGCTGGTGCTG
GAGCTGCTGTTGCTACACTTTTGGCTGTTTTCTCTCCTGGATTTGCTGCTTCAGCTATT
GGTATTGTTGCTGTTGGAGTTGCTATTGCTACTATTGCTTCTCTTTTGAACGCTGATAA
CGTTGAGAAGATTAATGTTTTTATTTCAGATCATTTGGAAACAGCTCTTAAAGAGCAAA
GGTAA
**SEQ ID NO: 23: Ppu259 second segment** 
AEAEAKRIDDYKRAVAYVADANKFILEKYGAKLHQVVMDLQKDVSGKKIRSYNEALRTFEQ
VRTNPNARLSPQDTRAVVDALNALDKATYMDSVNRLAKGFGVTGKIVQAHSVVEKAIIGFQ
DGNWKPLLLEFESIAAGAGAGLLVALIAPPVLAAFSFPPVIAVVATGLLVAGVAALLDAKTVE
KINDTIFTLVETTPAH
**SEQ ID NO: 24: Ppu259 second segment coding sequence** 
GCAGAGGCTGAGGCTAAGAGGATCGATGATTACAAGCGGGCTGTTGCTTACGTGGCC
GATGCTAACAAGTTCATCCTCGAGAAGTACGGTGCCAAGCTTCATCAGGTGGTGATGG
ATCTGCAGAAGGACGTTAGCGGCAAGAAAATCCGGTCTTACAACGAGGCTCTTAGGAC
CTTTGAGCAGGTCAGGACTAATCCTAACGCTAGGTTGTCTCCTCAGGACACTAGGGCT
GTTGTGGATGCTCTTAACGCTCTGGACAAGGCCACCTACATGGATTCTGTGAACAGGC
TGGCTAAAGGTTTCGGTGTGACCGGTAAGATTGTGCAGGCTCATTCTGTGGTCGAGAA
GGCTATTATCGGTTTCCAGGATGGCAACTGGAAGCCTCTTTTGCTCGAGTTCGAGTCT
ATTGCTGCTGGTGCTGGTGCAGGTTTGCTTGTGGCTCTTATTGCTCCTCCTGTGCTGG
CTGCTTTTTCTTTCCCACCTGTGATTGCTGTGGTGGCTACTGGTCTTTTGGTTGCTGGT
GTTGCTGCTCTGCTTGACGCTAAGACTGTCGAGAAGATCAACGACACCATCTTCACCC
TGGTTGAGACTACTCCTGCTCACTAA
**SEQ ID NO: 25: S5-Ppu259,** pyocin S5 part is underlined 
MSNDNEVPGSMVIVAQGPDDQYAYEVPPIDSAAVAGNMFGDLIQREIYLQKNIYYPVRSIF
EQGTKEKKEINKKVSDQVDGLLKQITQGKREATRQERVDVMSAVLHKMESDLEGYKKTFT
KGPFIDYEKQSSLSIYEAWVKIWEKNSWEERKKYPFQQLVRDELERAVAYYKQDSLSEAV
KVLRQELNKQKALKEKEDLSQLERDYRTRKANLEMKVQSELDQAGSALPPLVSPTPEQWL
ERATRLVTQAIADKKQLQTTNNTLIKNSPTPLEKQKAIYNGELLVDEIASLQARLVKLNAETT
RRRTEAEAEAKRIDDYKRAVAYVADANKFILEKYGAKLHQVVMDLQKDVSGKKIRSYNEAL
RTFEQVRTNPNARLSPQDTRAVVDALNALDKATYMDSVNRLAKGFGVTGKIVQAHSVVEK
AIIGFQDGNWKPLLLEFESIAAGAGAGLLVALIAPPVLAAFSFPPVIAVVATGLLVAGVAALLD
AKTVEKINDTIFTLVETTPAH
**SEQ ID NO: 26: S5-Ppu259 coding sequence**, pyocin S5 part is underlined 
ATGTCCAATGATAACGAAGTGCCAGGTTCAATGGTTATAGTTGCTCAAGGTCCAGATG
ATCAATACGCTTACGAAGTTCCTCCAATCGATTCTGCTGCTGTTGCTGGAAATATGTTC
GGAGATCTTATCCAAAGGGAAATATATCTTCAAAAGAATATCTATTACCCAGTTAGATCT
ATCTTCGAGCAAGGAACAAAGGAAAAGAAAGAGATTAATAAGAAAGTTTCAGATCAAGT
TGATGGACTTTTGAAGCAAATTACACAAGGAAAGAGAGAAGCTACTAGGCAAGAGAGA
GTTGATGTTATGTCAGCTGTTCTTCATAAGATGGAATCTGATTTGGAGGGATATAAGAA
AACTTTTACAAAGGGTCCTTTTATTGATTACGAAAAGCAATCTTCACTTTCAATCTATGA
AGCTTGGGTTAAGATTTGGGAGAAAAATTCTTGGGAGGAGAGAAAGAAATACCCTTTT
CAACAACTTGTTAGGGATGAATTGGAGAGAGCTGTTGCTTATTACAAGCAAGATTCTCT
TTCAGAAGCTGTTAAAGTTCTTAGGCAAGAGTTGAATAAGCAAAAGGCTTTGAAGGAAA
AGGAGGATCTTTCACAATTGGAAAGAGATTACAGGACAAGAAAGGCTAATCTTGAGAT
GAAGGTTCAATCTGAGTTGGATCAAGCTGGATCAGCTCTTCCACCTTTGGTTTCTCCAA
CTCCTGAACAATGGCTTGAGAGGGCTACAAGATTGGTTACTCAAGCTATTGCTGATAA
GAAACAACTTCAAACTACAAATAACACATTGATTAAGAACTCTCCAACTCCTCTTGAAAA
GCAAAAAGCTATCTATAATGGTGAACTTTTGGTTGATGAGATTGCTTCATTGCAAGCTA
GGCTTGTTAAATTGAATGCTGAGACTACAAGAAGGAGAACAGAAGCAGAGGCTGAGG
CTAAGAGGATCGATGATTACAAGCGGGCTGTTGCTTACGTGGCCGATGCTAACAAGTT
CATCCTCGAGAAGTACGGTGCCAAGCTTCATCAGGTGGTGATGGATCTGCAGAAGGA
CGTTAGCGGCAAGAAAATCCGGTCTTACAACGAGGCTCTTAGGACCTTTGAGCAGGTC
AGGACTAATCCTAACGCTAGGTTGTCTCCTCAGGACACTAGGGCTGTTGTGGATGCTC
TTAACGCTCTGGACAAGGCCACCTACATGGATTCTGTGAACAGGCTGGCTAAAGGTTT
CGGTGTGACCGGTAAGATTGTGCAGGCTCATTCTGTGGTCGAGAAGGCTATTATCGGT
TTCCAGGATGGCAACTGGAAGCCTCTTTTGCTCGAGTTCGAGTCTATTGCTGCTGGTG
CTGGTGCAGGTTTGCTTGTGGCTCTTATTGCTCCTCCTGTGCTGGCTGCTTTTTCTTTC
CCACCTGTGATTGCTGTGGTGGCTACTGGTCTTTTGGTTGCTGGTGTTGCTGCTCTGC
TTGACGCTAAGACTGTCGAGAAGATCAACGACACCATCTTCACCCTGGTTGAGACTAC
TCCTGCTCACTAA
SEQ ID NOs: 27-38: primer sequences shown in Suppl. Table 2
SEQ ID NO: 39: Pyo S5 second segment (killing domain)

aerkaaeeqal qdaikftadf ykevtekfga rtsemarqla egargknirs saeaiksfek hkdalnkkls lkdrqaiaka
fdsldkqmma kslekfskgf gvvgkaidaa slyqefkist etgdwkpffv kietlaagaa aswlvgiafa tatatpigil
gfalvmavtg amidedllek annlvisi

### References

1. Jurado-Martín, I., Sainz-Mejías, M. & McClean, S. Pseudomonas aeruginosa: An audacious pathogen with an adaptable arsenal of virulence factors. Int. J. Mol. Sci. 22, 1-37 (2021).
2. Lupo, A., Haenni, M. & Madec, J.-Y. Antimicrobial Resistance in Acinetobacter spp. and Pseudomonas spp. Microbiol. Spectr. 6, 316-318 (2018).
3. Olivares, E. et al. Clinical Impact of Antibiotics for the Treatment of Pseudomonas aeruginosa Biofilm Infections. Front. Microbiol. 10, 1-12 (2020).
4. Gillor, O., Kirkup, B. C. & Riley, M. A. Colicins and microcins: The next generation antimicrobials. Adv. Appl. Microbiol. 54, 129-146 (2004).
5. Michel-Briand, Y. & Baysse, C. The pyocins of Pseudomonas aeruginosa. Biochimie (2002). doi:10.1016/S0300-9084(02)01422-0
6. Ghequire, M. G. K. & De Mot, R. Ribosomally encoded antibacterial proteins and peptides from Pseudomonas. FEMS Microbiology Reviews (2014). doi:10.1111/1574-6976.12079
7. Scholl, D. & Martin, D. W. Antibacterial efficacy of R-type pyocins towards Pseudomonas aeruginosa in a murine peritonitis model. Antimicrob. Agents Chemother. (2008). doi:10.1128/AAC.01479-07
8. Brown, C. L., Smith, K., McCaughey, L. & Walker, D. Colicin-like bacteriocins as novel therapeutic agents for the treatment of chronic biofilm-mediated infection. Biochem. Soc. Trans. (2012). doi:10.1042/BST20120241
9. Smith, K. et al. Activity of pyocin S2 against Pseudomonas aeruginosa biofilms. Antimicrob. Agents Chemother. (2012). doi:10.1128/AAC.05714-11
10. McCaughey, L. C., Ritchie, N. D., Douce, G. R., Evans, T. J. & Walker, D. Efficacy of species-specific protein antibiotics in a murine model of acute Pseudomonas aeruginosa lung infection. Sci. Rep. (2016). doi:10.1038/srep30201
11. Paškevičius, S. et al. Plant-expressed pyocins for control of Pseudomonas aeruginosa. PLoS One 12, (2017).
12. Six, A. et al. Pyocin efficacy in a murine model of Pseudomonas aeruginosa sepsis. J. Antimicrob. Chemother. 76, 2317-2324 (2021).
13. Resch, G., Moreillon, P. & Fischetti, V. A. A stable phage lysin (Cpl-1) dimer with increased antipneumococcal activity and decreased plasma clearance. Int. J. Antimicrob. Agents 38, 516-521 (2011).
14. Hydrolases, L. P., Shen, Y., Zinkernagel, A. S. & Loessner, M. J. crossm Enables Efficient Treatment of Systemic Staphylococcus aureus. 11, 1-16 (2020).
15. Rasouliha, B. H., Ling, H., Ho, C. L. & Chang, M. W. A predicted immunity protein confers resistance to pyocin S5 in a sensitive strain of pseudomonas aeruginosa. ChemBioChem (2013). doi:10.1002/cbic.201300410
16. Ghequire, M. G. K., Kemland, L., Anoz-Carbonell, E., Buchanan, S. K. & De Mot, R. A natural chimeric Pseudomonas bacteriocin with novel pore-forming activity parasitizes the ferrichrome transporter. MBio (2017). doi:10.1128/mBio.01961-16
17. Behrens, H. M. et al. Pyocin S5 import into pseudomonas aeruginosa reveals a generic mode of bacteriocin transport. MBio 11, 1-16 (2020).
18. Choy, M. H., Stapleton, F., Willcox, M. D. P. & Zhu, H. Comparison of virulence factors in Pseudomonas aeruginosa strains isolated from contact lens- and noncontact lens-related keratitis. J. Med. Microbiol. 57, 1539-1546 (2008).
19. Fleiszig, S. M. J. et al. Relationship between cytotoxicity and corneal epithelial cell invasion by clinical isolates of Pseudomonas aeruginosa. Infect. Immun. 64, 2288-2294 (1996).
20. Finck-Barbançon, V. et al. ExoU expression by Pseudomonas aeruginosa correlates with acute cytotoxicity and epithelial injury. Mol. Microbiol. 25, 547-557 (1997).
21. Saliba, A. M. et al. Type III secretion-mediated killing of endothelial cells by Pseudomonas aeruginosa. Microb. Pathog. 33, 153-166 (2002).
22. Ajayi, T., Allmond, L. R., Sawa, T. & Wiener-Kronish, J. P. Single-nucleotide-polymorphism mapping of the Pseudomonas aeruginosa type III secretion toxins for development of a diagnostic multiplex PCR system. J. Clin. Microbiol. 41, 3526-3531 (2003).
23. Schmelcher, M. & Loessner, M. J. Bacteriophage endolysins - extending their application to tissues and the bloodstream. Curr. Opin. Biotechnol. 68, 51-59 (2021).
24. Roach, D. R. & Donovan, D. M. Antimicrobial bacteriophage-derived proteins and therapeutic applications. Bacteriophage 5, e1062590 (2015).
25. Briers, Y. et al. Engineered endolysin-based "Artilysins" to combat multidrug-resistant Gram-negative pathogens. MBio (2014). doi:10.1128/mBio.01379-14
26. Lukacik, P. et al. Structural engineering of a phage lysin that targets Gram-negative pathogens. Proc. Natl. Acad. Sci. U. S. A. 109, 9857-9862 (2012).
27. Heselpoth, R. D., Euler, C. W., Schuch, R. & Fischetti, V. A. Lysocins: Bioengineered antimicrobials that deliver lysins across the outer membrane of Gram-negative bacteria. Antimicrob. Agents Chemother. 63, (2019).
28. Parret., A. & De Mot., R. Novel bacteriocins with predicted tRNase and pore-forming activities in Pseudomonas aeruginosa PAO1. Mol. Microbiol. 35, 472-475 (2000).
29. Elfarash, A. et al. Pore-forming pyocin S5 utilizes the FptA ferripyochelin receptor to kill Pseudomonas aeruginosa. Microbiol. (United Kingdom) (2014). doi:10.1099/mic.0.070672-0
30. Snopkova, K. et al. Prevalence of bacteriocins and their co-association with virulence factors within Pseudomonas aeruginosa catheter isolates. Int. J. Med. Microbiol. 310, 151454 (2020).
31. Denkovskienė, E. et al. Broad and Efficient Control of Klebsiella Pathogens by Peptidoglycan-Degrading and Pore-Forming Bacteriocins Klebicins. Sci. Rep. 9, (2019).
32. Elhanan, M. M. & Nabi, A. Bacterial Keratitis Risk Factors, Pathogens and Antibiotic Susceptibilities: A 5- Year Review of Cases at Dubai hospital, Dubai. J. Clin. Exp. Ophthalmol. 07, (2016).
33. Willcox, M. D. P. Management and treatment of contact lens-related Pseudomonas keratitis. Clin. Ophthalmol. 6, 919-924 (2012).
34. Jean, S. S. et a/. Epidemiology, treatment, and prevention of nosocomial bacterial pneumonia. J. Clin. Med. 9, (2020).
35. Sadikot, R. T., Blackwell, T. S., Christman, J. W. & Prince, A. S. Pathogen-host interactions in pseudomonas aeruginosa pneumonia. Am. J. Respir. Crit. Care Med. 171, 1209-1223 (2005).
36. Planquette, B. et al. Pseudomonas aeruginosa ventilator-associated pneumonia: Predictive factors of treatment failure. Am. J. Respir. Crit. Care Med. 188, 69-76 (2013).
37. Maurice, N. M., Bedi, B. & Sadikot, R. T. Pseudomonas aeruginosa biofilms: Host response and clinical implications in lung infections. Am. J. Respir. Cell Mol. Biol. 58, 428-439 (2018).
38. Abdulwahab, A. et a/. The emergence of multidrug-resistant Pseudomonas aeruginosa in cystic fibrosis patients on inhaled antibiotics. Lung India 34, (2017).
39. Marillonnet, S., Thoeringer, C., Kandzia, R., Klimyuk, V. & Gleba, Y. Systemic Agrobacterium tumefaciens-mediated transfection of viral replicons for efficient transient expression in plants. Nat. Biotechnol. 23, 718-723 (2005).
40. Chen, K. et al. Stimulator of interferon genes promotes host resistance against Pseudomonas aeruginosa keratitis. Front. Immunol. 9, 1-14 (2018).

## Claims

1. An antibacterial protein comprising a polypeptide comprising a first polypeptide segment and, preferably contiguous thereto, a second polypeptide segment, said first polypeptide segment being selected from the following group (a-i) to (d-i), said second polypeptide segment being selected from the following group (a-i)' to (d-iii)':
group (a-i) to (d-i):
(a-i) the segment of the amino acid sequence of SEQ ID NO: 1, or
(b-i) a segment having at least 80%, more preferably at least 85%, even more preferably at least 90%, and even more preferably at least 95% sequence identity to the amino acid sequence of SEQ ID NO: 1, or
(c-i) a segment having at least 85%, preferably at least 90%, more preferably at least 95%, and most preferably at least 98 % sequence similarity to the amino acid sequence of SEQ ID NO: 1, or
(d-i) a segment having from 1 to 60, preferably from 1 to 45, more preferably from 1 to 30, and most preferably from 1 to 15 amino acid residue substitutions, additions, insertions and/or deletions compared to the amino acid sequence of SEQ ID NO: 1;
group (a-i)' to (d-iii)':
(a-i)' the segment of the amino acid sequence of SEQ ID NO: 3, or
(a-ii)' the segment of the amino acid sequence of SEQ ID NO: 7, or
(a-iii)' the segment of the amino acid sequence of SEQ ID NO: 11; or
(b-i)' a segment having at least 80%, more preferably at least 85%, even more preferably at least 90%, and most preferably at least 95% sequence identity to the amino acid sequence of SEQ ID NO: 3, or
(b-ii)' a segment having at least 80%, preferably at least 85%, more preferably at least 90%, and most preferably at least 95% sequence identity to the amino acid sequence of SEQ ID NO:7, or
(b-iii)' a segment having at least 80%, preferably at least 85%, more preferably at least 90%, and most preferably at least 95% sequence identity to the amino acid sequence of SEQ ID NO:11; or
(c-i)' a segment having at least 85%, preferably at least 90%, and more preferably at least 95%, and most preferably at most 98% sequence similarity to the amino acid sequence of SEQ ID NO: 3, or
(c-ii)' a segment having at least 85%, preferably at least 90%, and more preferably at least 95%, and most preferably at most 98% sequence similarity to the amino acid sequence of SEQ ID NO: SEQ ID NO: 7, or
(c-iii)' a segment having at least 85%, preferably at least 90%, and more preferably at least 95%, and most preferably at most 98% sequence similarity to the amino acid sequence of SEQ ID NO: SEQ ID NO: 11; or
(d-i)' a segment having from 1 to 38, preferably from 1 to 28, more preferably from 1 to 19, and most preferably from 1 to 10 amino acid residue substitutions, additions, insertions and/or deletions compared to the amino acid sequence of SEQ ID NO: 3, or
(d-ii)' a segment having from 1 to 38, preferably from 1 to 28, more preferably from 1 to 19, and most preferably from 1 to 10 amino acid residue substitutions, additions, insertions and/or deletions compared to the amino acid sequence of SEQ ID NO: 7, or
(d-iii)' a segment having from 1 to 38, preferably from 1 to 28, more preferably from 1 to 19, and most preferably from 1 to 10 amino acid residue substitutions, additions, insertions and/or deletions compared to the amino acid sequence of SEQ ID NO: 11.

2. An antibacterial protein comprising a polypeptide comprising or consisting of:
(A-i) the amino acid sequence of SEQ ID NO: 5, or
(A-ii) the amino acid sequence of SEQ ID NO: 9, or
(A-iii) the amino acid sequence of SEQ ID NO: 13; or
(B-i) an amino acid sequence having at least 80%, more preferably at least 85%, even more preferably at least 90%, and even more preferably at least 95% sequence identity to the amino acid sequence of SEQ ID NO: 5, or
(B-ii) an amino acid sequence having at least 80%, more preferably at least 85%, even more preferably at least 90%, and even more preferably at least 95% sequence identity to the amino acid sequence of SEQ ID NO: 9, or
(B-iii) an amino acid sequence having at least 80%, more preferably at least 85%, even more preferably at least 90%, and even more preferably at least 95% sequence identity to the amino acid sequence of SEQ ID NO: 13; or
(C-i) an amino acid sequence having at least 85%, preferably at least 90%, and more preferably at least 95%, and most preferably at least 98% sequence similarity to the amino acid sequence of SEQ ID NO: 5, or
(C-ii) an amino acid sequence having at least 85%, preferably at least 90%, and more preferably at least 95%, and most preferably at least 98% sequence similarity to the amino acid sequence of SEQ ID NO: 9, or
(C-iii) an amino acid sequence having at least 85%, preferably at least 90%, and more preferably at least 95%, and most preferably at least 98% sequence similarity to the amino acid sequence of SEQ ID NO: 13; or
(D-i) an amino acid sequence having from 1 to 100, preferably from 1 to 75, more preferably from 1 to 50, even more preferably from 1 to 25, and most preferably from 1 to 12 amino acid residue substitutions, additions, insertions and/or deletions to the amino acid sequence of SEQ ID NO: 5, or
(D-ii) an amino acid sequence having from 1 to 100, preferably from 1 to 75, more preferably from 1 to 50, even more preferably from 1 to 25, and most preferably from 1 to 12 amino acid residue substitutions, additions, insertions and/or deletions to the amino acid sequence of SEQ ID NO: 9; or
(D-iii) an amino acid sequence having from 1 to 100, preferably from 1 to 75, more preferably from 1 to 50, even more preferably from 1 to 25, and most preferably from 1 to 12 amino acid residue substitutions, additions, insertions and/or deletions to the amino acid sequence of SEQ ID NO: 13.

3. A composition comprising a protein according to claim 1 or 2.

4. The composition according to claim 3, further comprising a second bacteriocin according to claim 1 or 2.

5. A pharmaceutical composition comprising an antibacterial protein as defined in any one of claims 1 and 2, or comprising a composition according to any one of claims 3 and 4, and one or more pharmaceutically acceptable carrier(s) or excipient(s).

6. The pharmaceutical composition according to claim 5, which is a sterile aqueous solution comprising said antibacterial protein or is a solid formulation, preferably said solid formulation is a powder suitable for reconstitution in an aqueous liquid medium or is a solid formulation suitable for administration as an aerosol.

7. The antibacterial protein according to any one of claims 1 and 2, or a composition according to any one of claims 3 to 6 for use in therapy.

8. The antibacterial protein according to any one of claims 1 and 2, or a composition according to any one of claims 3 to 6, for use in the treatment or prevention of a bacterial infection, preferably of a bacterial infection by *Pseudomonas,* more preferably by *P. aeruginosa.*

9. The antibacterial protein according to any one of claims 1 and 2, or a composition according to any one of claims 3 to 6, for use in therapy, preferably for the treatment of lung infection or keratitis.

10. The antibacterial protein according to any one of claims 1 and 2, or a composition according to any one of claims 3 to 6, for use in the treatment of lung infection by administration into the lungs of a mammal as a solid or liquid aerosol; or for the treatment of keratitis by administration into an affected eye of a mammal in the form of an aqueous solution.

11. A method of treating or preventing bacterial infection in a mammal, comprising administering to a mammal in need thereof an antibacterial protein as defined in any one of claims 1 and 2, or a composition according to claim 3 or 4, or a pharmaceutical composition according to claim 5 or 6.

12. The method according to claim 11, wherein bacterial lung infection is treated by administration into the lungs of a mammal as a solid or liquid aerosol; or wherein bacterial keratitis is treated by administration into an affected eye of a mammal in the form of an aqueous solution.

13. A nucleic acid molecule comprising a nucleic acid sequence encoding a protein or a polypeptide according to any one of claims 1 and 2.

14. The nucleic acid molecule according to claim 13, said nucleic acid sequence comprising a polynucleotide according to any one of SEQ ID NOs: 6, 10, or 14, or a polynucleotide encoding a polypeptide as defined in claim 2.

15. Bacterial or eukaryotic cell comprising a nucleic acid molecule according to claim 13 or 14.
